# EUROPÄISCHE PATENTSCHRIFT

(11) **EP 4 437 932 B1**
(45) Veröffentlichungstag und Bekanntmachung des Hinweises auf die Patenterteilung: **29.10.2025**
(21) Anmeldenummer: 24187397.5
(22) Anmeldetag: 20.11.2020
(51) Int. Cl.: A61B 1/06, A61B 1/07

(54) **LICHTQUELLE FÜR ENDOSKOP**
LIGHT SOURCE FOR ENDOSCOPE
SOURCE LUMINEUSE POUR UN ENDOSCOPE

(30) Priorität: 04.12.2019 DE 102019133042
(43) Veröffentlichungstag der Anmeldung: 02.10.2024
(62) Teilanmeldung aus: 20209014.8
(73) Patentinhaber: SCHOTT AG, 55122 Mainz (DE)
(72) Erfinder: SCHULTHEIS, Bernd, 55270 Schwabenheim (DE); CRAMER, Martin, 65187 Wiesbaden (DE); RUSSERT, Hubertus, 55270 Jugenheim (DE); WERNER, Holger, 60596 Frankfurt am Main (DE); VIETOR, Jens, 65232 Taunusstein (DE); HAGEMANN, Volker, 55268 Nieder-Olm (DE); MEINL, Jürgen, 65329 Hohenstein-Holzhausen (DE); GRIMM, Jonas, 65307 Bad Schwalbach (DE); KEIPER, Oliver, 65510 Hünstetten (DE)
(74) Vertreter: Blumbach · Zinngrebe Patentanwälte PartG mbB

(56) Entgegenhaltungen:
- EP-A1- 3 097 845
- DE-A1- 102006 053 487
- US-A1- 2012 010 465
- US-A1- 2018 228 354

## Beschreibung

### Gebiet der Erfindung

Die Erfindung betrifft allgemein eine Lichtquelle für ein Endoskop und/oder für ein Endoskopsystem, beispielsweise für ein Einweg-Endoskop und/oder ein Einweg-Endoskopsystem.

### Hintergrund der Erfindung

Diagnose-, Operations- und/oder Therapiegeräte, wie beispielsweise Endoskope zur Diagnose, für minimalinvasive Eingriffe oder zur Therapie, sind als starre oder flexible Ausführungen bekannt und in der Literatur hinreichend beschrieben. Einweg-Endoskope, oder auch "disposable Endoscopes" genannt, werden heute zunehmend eingesetzt, um insbesondere die Patientensicherheit bei medizintechnische Untersuchungen, Therapien und/ oder minimal invasive Eingriffen zu erhöhen, in dem durch eine Einmal-Verwendung Kontaminationen verhindert werden. Zwar sind bisherige Endoskope dafür konzipiert, dass diese im Sinne der Medizintechnik aufbereitbar, das heißt reinigbar, sterilisierbar und vor allem autoklavierbar, sind.

Trotzdem kann es hier aufgrund falscher Anwendung der Aufbereitung bzw. ungünstigem Design derartiger Geräte vereinzelt vorkommen, dass nicht die erforderliche Keimzahlreduzierung erreicht wird, und damit Keime bei der nächsten Anwendung auf den Patienten übertragen werden können. Dies kann durch den Einsatz von derartigen Einweg-Endoskopen verhindert werden.

Ein weiterer Aspekt für den vermehrten Einsatz von Einweg-Endoskopen stellt auch eine Wirtschaftlichkeitsbetrachtung dar. Insbesondere der ordnungsgemäß und regelmäßig nach jeder Behandlung durchgeführte Aufbereitungsprozess erfordert inzwischen hohe Kosten beim praktizierenden Arzt oder in der Klinik. Zudem sind hohe Investitionen für Reinigungsgeräte, wie Thermodesinfektoren, und Autoklaviergeräten und/ oder Plasma-Sterilisationsgeräte erforderlich, so dass insgesamt der Einsatz derartiger Einweg-Endoskope gerechtfertigt ist.

Ein weiterer Vorteil ergibt sich daraus, dass derartige Einweg-Endoskope zum einen mobil als "hand-held"-Geräte einsetzbar sind und daher auch in der Notfall-Medizin, im Militär-Sanitätseinsatz oder auch in schwer zugänglichen Regionen, zum Beispiel auch bei Katastropheneinsätzen, einsetzbar sind, wo insbesondere keine Aufbereitungsmöglichkeiten zur Verfügung stehen.

Derartige Einweg-Endoskope, "Single-Use"-Endoskope oder "disposable Endoscopes", wie sie in der Literatur beschrieben sind, sind beispielhaft in folgenden Schriften beschrieben:
Die Schrift US 3581738 A1 offenbart ein Einweg-Endoskop, umfassend einen Körper aus synthetischem Harzmaterial mit einer im allgemeinen rohrförmigen Seitenwand, die ein Spekulum bildet, und ein einheitliches längliches Lichtleitelement, das in die Seitenwand eingebettet ist, wobei das Element aus einem Lichtleitmaterial gebildet ist, das mit einem transparenten Material mit einem Brechungsindex, der sich von dem des Lichtleitmaterials unterscheidet, überzogen ist, wobei der Körper aus zwei sich axial vom Endoskop geteilten Paarungshälften gebildet ist, wobei jede Hälfte ein Elementumschließungselement aufweist.

In der Schrift US 4964710 A1 wird ein starres Endoskop beschrieben, welches mit einem Objektivsystem, einer Okularlinse und einer Zwischenrelaislinse ausgestattet. Das Relaissystem ist ein Hybridsystem, das sowohl Kunststoff- als auch Glaselemente verwendet. Die Kunststoffelemente bestehen aus einer geradzahligen Anzahl (N) axial ausgerichteter Linsen, die jeweils eine Länge in der gleichen Größenordnung wie ihr Durchmesser aufweisen. Die Glaselemente sind eine ungeradzahlige Anzahl (N minus 1) von axial ausgerichteten Glas-Planzylindern, deren Stirnflächen poliert sind.

Die Schrift EP 1890173 A1 beschreibt eine Methode zur Herstellung eines Lichtleiters, wie er in derartigen Endoskopen einsetzbar ist. Dabei werden eine Vielzahl von optischen Fasern gebündelt und anschließend das Faserbündel an einem Teil eines Mundstücks geschnitten, das an einem Zwischenteil des Faserbündels befestigt ist. So wird das Faserbündel in ein erstes optisches Faserbündel und ein zweites optisches Faserbündel aufgeteilt. Die Teilungsflächen des ersten und zweiten optischen Faserbündels haben die gleichen Eigenschaften und Bedingungen, da die ersten und zweiten optischen Faserbündel aus dem Faserbündel gebildet sind, das durch Bündelung der gleichen optischen Fasern erhalten wird. Das erste optische Faserbündel ist in einem Einführungsabschnitt eines Endoskops montiert und das zweite optische Faserbündel ist in einem flexiblen Schlauch montiert, womit ein erster Lichtleiter in dem Einführungsabschnitt des Endoskops und ein zweiter Lichtleiter in dem flexiblen Schlauch ausgebildet sind. Dadurch entsteht eine trennbare Lichttransmissionsstrecke des Lichtleiters.

Da aufgrund ihrer Einmal-Verwendung derartige Endoskope unter einem hohen Kostendruck stehen, müssen die Baugruppen bzw. Komponenten kostenoptimiert herstellbar sein. Eine der Hauptkomponenten zur Bildgebung und Beleuchtung stellen Lichtleiter oder Bildleiter dar. Diese werden derzeit noch in vergleichsweise aufwendigen Prozessschritten montiert bzw. bearbeitet. Oft sind es komplexe mechanische Komponenten, teils kombiniert mit optischen Elementen, wie Linsen, die diese Licht- bzw. Bildleiter beinhalten, und teils sind es auch aufwendige Bearbeitungsschritte, wie das Schleifen und Polieren der Endfläche, die derzeitige Lichtleiter bzw. Bildleiter vergleichsweise kostenaufwendig machen.

Andererseits müssen aber auch noch gewisse lichttechnische Anforderungen beim endoskopischen Einsatz insbesondere in der Medizintechnik berücksichtigt werden. Dies sind neben möglichst verlustfreier Bereitstellung des von einer Lichtquelle bereitgestellten Lichtes an den Untersuchungsort, farbtreue bzw. gezielt farbige Darstellung des Untersuchungsortes auch die Vermeidung unnötige Wärme an den Untersuchungsort einzubringen. Eine besondere Herausforderung stellt dabei der von der Lichtquelle zur Verfügung gestellte Lichtstrom und die Weiterleitung des Lichtes zum distalen Ende eines Endoskops dar. Insbesondere für Endoskopsysteme mit kleinem Durchmesser bedarf es extrem heller Lichtquellen zum einen und Lichtstrom-optimierte Lichtleiter zum anderen.

Bei Verwendung von aktiven elektronischen Bauelementen, wie beispielsweise Kamera-Chips und/ oder LED zur Beleuchtung müssen zudem noch Anforderungen hinsichtlich elektrischer Isolation, elektrischer Abschirmung, sowie Patientenableitströmen berücksichtigt werden, die je nach Einsatzgebiet des Endoskops maximale Grenzwerte nicht überschreiten dürfen. So ist beispielsweise bei Anwendungen am Herzen ein max. Ableitstrom von 10 µA gefordert, was einer CF-Klassifizierung entspricht (vergl. EN 60601-1, 3. Ausgabe Tab. 3).

Neben diesen lichttechnischen und elektrischen Anforderungen sind auch noch Anforderungen hinsichtlich der Biokompatibilität zu beachten. Für die Biokompatibilität ist es erforderlich sicherzustellen, dass das Material mit menschlichem Organismus verträglich ist. Für Medizinprodukte, die in Kontakt zum menschlichen Körper kommen können, ist es regulatorisch gefordert, mögliche Interaktionen und unerwünschte Nebenwirkungen zu bestimmen und zu bewerten. Die Wahl der erforderlichen Prüfungen ergibt sich aus der Kontaktart und der Kontaktdauer im menschlichen Körper. Entsprechend der europäischen Richtlinie für Medizinprodukte MDD 93/42 EWG (kurz MDD) bzw. der Verordnung (EU) 2017/745 vom 5. April 2017 (kurz MDR) ist diese biologische Beurteilung eines Produktes immer dann notwendig, wenn ein unmittelbarer Kontakt von Werkstoff/Produkt mit dem Patienten besteht.

Die Hauptregelwerke zur biologischen Untersuchung und Beurteilung von Werkstoffen sind die DIN EN ISO 10993 und die Prüfung nach United States Pharmacopeia Class VI (USP Class VI). Obwohl die deutlich umfangreichere ISO 10993 ursprünglich den Test nach USP Class VI ersetzen sollte, wird die USP-Prüfung heute insbesondere sehr häufig zur Beurteilung von biokompatiblen Kunststoffen herangezogen. Dazu werden die für eine invasive Applikation vorgesehenen Materialien zum einen hinsichtlich ihrer chemischen Verbindung bewertet und zum anderen einem Zytotoxizitätstest unterzogen, bei dem möglichen toxischen Wirkungen auflebende Zellkulturen untersucht werden. Die Anforderungen hierfür sind in der DIN EN ISO 10993, insbesondere in den Teilen -1 und -5 zusammengefasst (DIN EN ISO 10993-1: 2010-04). In USA unterliegt dies den Anforderungen der FDA. Zur DIN EN ISO 10993 korrespondierende Anforderungen sind dort in der USP Class VI hinterlegt.

Weiterhin kommt der Auslegung der Endoskope als Einweg-Endoskope zu Gute, dass die als Aufbereitungsmethoden bekannten Reinigungs-/ Desinfektionsverfahren mit stark basischen Lösungen und die Sterilisation mittels Autoklavieren bei Temperatur bis zu 135° C und typischen Dampfdrücken von etwa 3 bar bei der Materialauswahl nicht in diesem Maße berücksichtigt werden müssen, was insbesondere auch eine kostengünstigere Materialauswahl erlaubt. Lediglich sind die Eignung für Gas-Sterilisationsverfahren, wie die Ethylenoxid-Sterilisation, sowie RoHS- als auch REACH-Bestimmungen bei den Materialien zu berücksichtigen.

Die eigenen Anmeldungen der Anmelderin mit den Aktenzeichen DE 10 2019 125 912 sowie DE 10 2018 107 523 befassen sich mit verschiedenen Aspekten der Lichtleiter. Eine laserbasierte Lichtquelle wird nicht genannt.

Das US-amerikanische Patent US 6 398 721 B betrifft eine chirurgische Mikroskopievorrichtung, welche eine Laserdiode umfassen kann.

Die US-amerikanische Patentanmeldung US 2006/0279950 A1 beschreibt eine LED. Endoskope werden nicht genannt, allerdings können beispielsweise Lichtleiter umfassend Fasern verwendet werden. Die LED wird in Transmission betrieben.

Ebenso beschreibt auch die US-amerikanische Patentanmeldung US 2006/0152926 A1 eine LED, welche beispielsweise auch in Endoskopen verwendet werden kann. Die LED wird in Transmission betrieben.

In der US-amerikanischen Patentschrift US 5 436 655 A wird ein Endoskop beschrieben, welches einen Laser umfassen kann.

Eine hocheffiziente Lichtquelle ist in der US-Patentanmeldung US 2004/0246744 A1 beschrieben.

In der US-amerikanischen Patentanmeldung US 2019/0014979 A1 wird ein Endoskop beschrieben, welches auch mit Laserlicht betrieben werden kann.

Die US-amerikanische Patentanmeldung US 2019/0290100 A1 beschreibt ein optisches Abbildungssystem, welches insbesondere in der Fluoreszenzmikroskopie (STED-Mikroskopie) Verwendung finden kann.

In der internationalen Patentanmeldung WO 2013/092498 A1 wird ein Endoskop beschrieben, welches Laserdioden als Lichtquellen umfassen kann.

Die US-amerikanische Patentanmeldung US 2006/0069314 A1 beschreibt eine Festkörperlichtquelle für ein Endoskop.

In der deutschen Patentanmeldung DE 10 2017 108 698 A1 wird ein optoelektronisches Bauelement beschrieben.

Die US-amerikanische Patentanmeldung US 2018/0228354 A1 betrifft eine Lichtquelle für ein Endoskop sowie das Endoskop selbst, nennt jedoch keine Glasfasern als Lichtleiter.

Die US-amerikanische Patentanmeldung US 2012/010465 A1 betrifft ebenfalls ein Endoskop, nennt jedoch auch keine Glasfasern als Lichtleiter.

Die deutsche Patentanmeldung DE 10 2006 053 487 A1 betrifft ein Endoskopsystem mit fasergepumpter Fluoreszenzbeleuchtung.

Die europäische Patentanmeldung EP 3 097 845 A1 betrifft ein Lichtquellenmodul und ein Lichtquellensystem für ein Endoskop.

Schließlich betrifft die US-amerikanische Patentanmeldung US 2019/038120 A1 eine medizinische Beleuchtungseinrichtung und damit verbundene Verfahren.

Es ist jedoch bisher nicht möglich, die Vorteile einer Beleuchtung mit hoher Leuchtdichte kostengünstig zu realisieren. Insbesondere ist es derzeit nicht möglich, die Vorteile von Laserlicht für Einweg-Endoskope zu realisieren.

### Aufgabe der Erfindung

Die Aufgabe der Erfindung besteht darin, die Schwächen des Standes der Technik wenigstens teilweise zu überwinden oder zumindest zu mindern, insbesondere aber eine Lichtquelle, beispielsweise für Endoskopsysteme, bereitzustellen, insbesondere für den Single-Use-Einsatz eine helle Lichtquelle bzw. eine Beleuchtung mit großer Helligkeit und ein dazu optimiertes Lichtleitsystem.

### Zusammenfassung der Erfindung

Die Aufgabe der Erfindung wird gelöst durch den Gegenstand der unabhängigen Ansprüche. Spezielle und bevorzugte Ausführungsformen finden sich in den abhängigen Ansprüchen.

Die Offenbarung betrifft daher eine Lichtquelle, insbesondere für ein Endoskop mit einem ersten Bauteil und einem zweiten Bauteil, wobei im ersten Bauteil eine Lichtquelle eingebaut ist, und wobei das zweite Bauteil ein mit dem ersten Bauteil verbundenes proximales Ende, vorzugsweise ein lösbar verbundenes proximales Ende, und ein distales Ende aufweist, wobei im distalen Ende ein Element zur Bildaufnahme oder -leitung und/oder Aufnahme oder Leitung von optischen Informationen wie beispielsweise ein Kamerachip oder ein faseroptisches Element angeordnet ist, und wobei im zweiten Bauteil ein Lichtleiter mit zumindest einer lichtleitenden Faser verläuft, um Licht der Lichtquelle vom proximalen Ende zum distalen Ende zu leiten und am distalen Ende abzugeben, sowie vorzugsweise eine Versorgungsleitung zur elektrischen Speisung des Kamerachips, insbesondere für den Fall, dass ein solcher Kamerachip am distalen Ende angeordnet ist, und wobei die Lichtquelle mindestens einen Laser zur Abgabe von Primärlicht umfasst, sowie einen Konverter, welcher das Licht des Lasers zumindest teilweise in Licht einer anderen Wellenlänge (Sekundärlicht) umwandelt und abgibt, wobei der Konverter so mit dem am ersten Bauteil verbundenen proximalen Ende des zweiten Bauteils gekoppelt ist, dass das vom Konverter umgewandelte und abgegebene Licht in den Lichtleiter eingekoppelt wird. Faseroptische Elemente zur Bildaufnahme bzw. Bildweiterleitung werden auch als "Image Guides" bezeichnet und bestehen aus einigen Zehntausend Einzelfasern, welche geordnet zueinander an den Endflächen angeordnet sind. Solche faseroptischen Elemente können insbesondere aus Glas oder aus Kunststoff bestehen oder Glas oder Kunststoff umfassen, beispielsweise als Glasfaser bzw. als Kunststofffaser ausgestaltet sein.

Eine solche Ausgestaltung einer Lichtquelle und eines damit ausgerüsteten Endoskops weist eine Reihe von Vorteilen auf.

Gemäß der vorliegenden Offenbarung ist das Endoskop nämlich in zwei Bauteile aufgeteilt. In das erste Bauteil, welches auch als proximales Bauteil bezeichnet werden kann, ist eine Lichtquelle eingebaut, welche mindestens einen Laser, der zur Abgabe von Primärlicht ausgebildet ist, umfasst. Beispielsweise kann der Laser so ausgestaltet sein, dass er zur Abgabe von blauem und/oder ultravioletten Licht ausgebildet ist. Weiterhin unterfasst das erste Bauteil einen Konverter, welcher ausgebildet ist, das Licht des Lasers zumindest teilweise in Licht einer anderen Wellenlänge umzuwandeln und abzugeben.

Dies ist vorteilhaft, weil auf diese Weise Laserlicht nutzbar ist. Insbesondere ist es auf diese Weise möglich, eine besonders hohe Beleuchtungsintensität zu erzielen.

Der Konverter ist dabei mit dem am ersten Bauteil verbundenen proximalen Ende des zweiten Bauteils gekoppelt, so dass das vom Konverter umgewandelte und abgegebene Licht in den Lichtleiter eingekoppelt wird bzw. einkoppelbar ist.

Mit anderen Worten ist das erste Bauteil so ausgestaltet, dass es mit einem zweiten Bauteil, welches auch als distales Bauteil bezeichnet werden kann, verbindbar ausgestaltet ist, oder es liegt sogar mit dem zweiten Bauteil verbunden vor.

Das erste Bauteil kann je nach genauer Ausgestaltung und je nach der Art der von ihm umfassten Elemente beispielsweise als Handstück ausgestaltet sein, also beispielsweise als ein Bauteil, welches auch dazu dient, das Endoskop zu handhaben und/oder zu halten. Es ist aber auch möglich, dass das erste Bauteil Elemente umfasst, welche zur Steuerung und/oder zum Betrieb eines Endoskops dienen, beispielsweise also als Steuer- und/oder Auswerteeinheit ausgestaltet sind, sodass in diesem Fall das erste Bauteil auch als ein Betriebsgerät für das Endoskop ausgestaltet sein kann.

Weiterhin umfasst das Endoskop ein zweites Bauteil, welches ein proximales und ein distales Ende aufweist und wobei im zweiten Bauteil ein Lichtleiter, der zumindest eine lichtleitende Faser umfasst, verläuft. Der Lichtleiter ist ausgestaltet, um Licht der Lichtquelle vom proximalen Ende zum distalen Ende zu leiten und am distalen Ende abzugeben. Am distalen Ende ist ein Element zur Bildaufnahme, wie ein Kamerachip zur Bildaufnahme oder ein faseroptischer Bildleiter, angeordnet. Weiterhin umfasst das zweite Bauteil vorzugsweise für den Fall, dass das distale Ende einen Kamerachip umfasst, eine Versorgungsleitung zur elektrischen Speisung des Kamerachips.

Eine solche Ausgestaltung des Endoskops mit zwei Bauteilen (oder auch Baugruppen) ist vorteilhaft. Denn das Endoskop ist gemäß der beschriebenen Ausgestaltung so aufgebaut, dass das erste Bauteil Elemente umfasst, wie beispielsweise die Lichtquelle umfassend mindestens einen Laser, welche relativ kostenintensiv sind, und das zweite Bauteil hingegen solche Elemente umfasst, die relativ kostengünstig sind. Es ist also möglich, das Endoskop aufzutrennen, und auf diese Weise können beispielsweise preiswerte Elemente in einer vergleichsweise preiswerten Single-Use-Baugruppe untergebracht werden, wohingegen die weniger preiswerten, kostenintensiven Elemente in einer Multi-Use-Baugruppe untergebracht sind.

Damit ist es nun erstmal möglich, beispielsweise ein Endoskop bereitzustellen, dass die Vorteile einer sehr hochwertigen Beleuchtung mit den Vorteilen eines lediglich für den einmaligen Gebrauch vorgesehenen Endoskops zu verbinden. Beachtlich ist hierbei, dass das Endoskop nach der vorliegenden Offenbarung aber nicht zwingend als Einmal-Endoskop bzw. zumindest teilweise als Einmal-Endoskop ausgestaltet sein muss. Vielmehr ist es auch denkbar, dies bedarfsweise anzupassen.

Es kann jedoch von Vorteil sein, wenn erste Bauteil und zweites Bauteil so miteinander verbunden sind, dass sie voneinander lösbar sind. Sofern das Endoskop als zumindest teilweise für den Einzelgebrauch vorgesehenes Endoskop ausgebildet ist, kann beispielsweise das zweite Bauteil nach Gebrauch entsorgt werden. Es ist aber auch möglich, dass das zweite Bauteil zwar lösbar mit dem ersten Bauteil verbunden ist, aber dennoch für den mehrfachen Gebrauch vorgesehen ist, und nach der Trennung vom ersten Bauteil bestimmten, für den medizinischen Gebrauch vorgesehenen Reinigungs- und Sterilisationsprozessen unterzogen wird.

Mit dem Endoskop nach der vorliegenden Offenbarung, welches auch als modulares Endoskop beschrieben werden kann, ist also zum einen auch die Möglichkeit einer vereinfachten Handhabung verbunden. Zum anderen kann, gerade wenn es sich um ein Einweg-Endoskop oder ein zumindest teilweise als Einweg-Endoskop ausgebildetes Endoskop handelt, also um ein Endoskop, von dem zumindest einige Bestandteile nur für den einmaligen Gebrauch bestimmt sind, eine hochwertige Beleuchtung, wie beispielsweise die Beleuchtung mittels eines Lasers, die eine hohe Lichtintensität ermöglicht, mit den Vorzügen eines Einweg-Geräts verbunden werden.

Das zweite Bauteil, welches einen Lichtleiter umfasst, kann beispielsweise starr ausgeführt sein, oder auch flexibel. Allgemein kann das zweite Bauteil auch als sogenannter Schaft eines Endoskops verstanden werden, wobei im Rahmen der vorliegenden Offenbarung unter einem Schaft allgemein sowohl ein starres zweites Bauteil verstanden wird als auch ein flexibles Bauteil, welches beispielsweise nur einen flexiblen äußeren Hüllschlauch beispielsweise umfassend ein Kunststoffmaterial umfasst. Sofern das zweite Bauteil starr ausgeführt ist, kann es beispielsweise so ausgestaltet sein, dass der Lichtleiter, welcher vom zweiten Bauteil umfasst wird, zumindest abschnittsweise von einem Rohrabschnitt oder von mehreren Rohrabschnitten umfassend ein Metall oder einen Kunststoff umgeben ist. Die genaue Ausführung des zweiten Bauteils kann dabei je nach bevorzugtem Einsatzgebiet des Endoskops ausgewählt sein.

Gemäß einer bevorzugten Ausführungsform umfasst der Konverter ein keramisches Konvertermaterial. Eine solche Ausgestaltung ist vorteilhaft, da damit eine besonders hohe Lichtintensität auch für weißes Licht möglich ist. Keramische Konvertermaterialien sind nämlich besonders temperaturstabil, so dass damit besonders hohe Leuchtdichten erzielt werden können. Denkbar sind auch organisch basierte Konverter oder Kombinationen aus organischen und keramischen Konvertermaterialien. Insbesondere ist es möglich, dass der Konverter so ausgestaltet ist, dass er ein Konverterelement umfasst, welches zwei oder mehr Konvertermaterialien umfasst, welche insbesondere so ausgestaltet sein können, dass sie Primärlicht in Licht unterschiedlicher spektraler Zusammensetzung konvertieren. Beispielsweise ist es denkbar, dass ein Konverterelement einen sogenannten "gelben" und einen sogenannten "roten" Phosphor umfasst. Unter einem Phosphor wird hier ein Leuchtstoff verstanden. Beispielsweise können diese Materialien als Mischung vorliegen, beispielsweise als Mischung umfassend ein organischen und ein keramisches Material, oder als Mischung aus organischen oder keramischen Materialien. Der Konverter kann aber auch so ausgestaltet sein, dass er mehrere Konverterelemente umfasst, welche jeweils ein unterschiedliches Konvertermaterial umfassen. Auch Mischungen dieser Ausführungen sind vorstellbar.

Insbesondere kann das keramische Konvertermaterial ein lumineszierendes keramisches Material sein oder ein solches umfassen. Im Rahmen der vorliegenden Offenbarung bedeutet dies, dass der Konverter beispielsweise überwiegend, also zu mindestens 50 Gew.-%, oder auch im Wesentlichen, also zu mindestens 90 Gew.-%, aus einem lumineszierenden keramischen Material bestehen kann. Auch ist es möglich, dass der Konverter vollständig aus dem lumineszierenden, keramischen Material besteht. Insbesondere umfasst also der Konverter und/oder das Konverterelement ein lumineszierendes keramisches Material oder besteht aus diesem. Der Konverter und/oder das Konverterelement kann auch als Kompositwerkstoff, beispielsweise als Phosphor-Glas-Komposit, oder als Phosphor-Kunststoff-Komposit, insb. Phosphor-Silikon-Komposit, oder als Phosphor-Keramik-Komposit ausgebildet sein und umfasst in diesem Fall vorzugsweise mindestens 10 Gew.-% eines lumineszierenden keramischen Materials, beispielsweise zwischen 10 Gew.-% und 30 Gew.-%, insbesondere zwischen 10 Gew.-% und 20 Gew.-%.

Gemäß einer Ausführungsform umfasst der Konverter und/oder das Konverterelement als lumineszierendes keramisches Material ein granatartiges keramisches Material oder besteht überwiegend, also zu mindestens 50 Gew.-%, oder im Wesentlichen, also zu mindestens 90 Gew.-%, oder vollständig aus diesem, wobei das granatartige keramische Material vorzugsweise die folgende Summenformel aufweist:

A₃B₅O₁₂:RE,

wobei
A Y und/oder Gd und/oder Lu sowie
B Al und/oder Ga
umfasst und wobei RE ausgewählt ist aus der Gruppe der Seltenen Erden und bevorzugt Ce und/oder Pr umfasst.

Gemäß einer nochmals weiteren Ausführungsform weist das granatartige keramische Material die folgende Summenformel auf:

(Y₁₋ₓCeₓ)₃Al₅O₁₂

und/oder

(Y_{1-x-y}Gd_{y}Ceₓ)₃Al₅O₁₂

und/oder

(Lu₁₋ₓCeₓ)₃Al₅O₁₂

und/oder

(Y_{1-x-z}Lu_{z}Ceₓ)₃Al₅O₁₂,

wobei für x jeweils gilt: 0,005 < x < 0,05
und wobei für y gilt: 0 < y < 0,2, und
wobei für z gilt: 0 < z < 1.

Gemäß einer Ausführungsform umfasst der Konverter und/oder das Konverterelement ein lumineszierendes keramisches Material oder besteht überwiegend, also zu mindestens 50 Gew.-%, oder im Wesentlichen, also zu mindestens 90 Gew.-%, oder vollständig aus diesem, wobei der Konverter
- als einphasige massive Keramik (also eine sogenannte Optokeramik) vorliegt und/oder
- als mehrphasige massive Keramik vorliegt und/oder
- als einphasige oder mehrphasige Keramik bestimmter Porosität vorliegt und/oder
- als Kompositwerkstoff vorliegt, wie als Phosphor-Glas-Komposit (engl.: phosphor in glass, PIG) und/oder als Phosphor-Silikon-Komposit (engl.: phosphor in silicone, PIS).

Gemäß einer weiteren Ausführungsform umfasst das keramische Material auch andere oxidische Verbindungen (außer Granatverbindungen), sowie auch nitridische Verbindungen, insbesondere aus der Gruppe der Aluminiumoxinitride und Siliziumaluminiumoxinitride.

Gemäß einer weiteren Ausführungsform ist der Konverter und/oder das Konverterelement als poröse Sinterkeramik ausgebildet und die Porosität liegt zwischen 0,5% und 10%, bevorzugt zwischen 4% und 8%. Die Porosität bezieht sich hierbei auf das Volumen. Vorzugsweise liegt die mittlere Porengröße zwischen 400 µm und 1200 µm, bevorzugt zwischen 600 µm und 1000 µm und besonders bevorzugt zwischen 600 µm und 800 µm.

Im Rahmen der vorliegenden Offenbarung wird unter einer einphasigen Keramik (oder Optokeramik) verstanden, dass wenigstens 95 Vol.-% der von der Keramik umfassten Kristalle und/oder Kristallite dieselbe Kristallphase sind. Vorzugsweise ist der Volumenanteil von Fremdphasen deutlich geringer. Insbesondere können sogar mehr als 96 Vol.-% oder mehr als 97 Vol.-% oder mehr als 98 Vol.-% oder sogar mehr als 99 Vol.-% der von der Keramik umfassten Kristalle und/oder Kristallite dieselbe Kristallphase ausbilden. Nicht ausgeschlossen ist weiterhin, dass eine einphasige Keramik noch amorphe Bestandteile umfassen kann. Diese liegen aber in der Regel bei weniger als 5 Vol.-%.

Besonders vorteilhaft kann es sein, wenn das keramische Material so ausgestaltet ist, dass das Material eine Wärmeleitfähigkeit im Bereich von 1 W/mK bis 20 W/mK aufweist. Auf diese Weise ist eine besonders gute Ableitung der bei der Konversion entstehenden bzw. entstandenen thermischen Energie möglich, sodass die Konversionseigenschaften des Konvertermaterials sich während des Betriebs des Materials nur geringfügig, wenn überhaupt, ändern.

Insbesondere kann das keramische Konvertermaterial polykristallin ausgestaltet sein.

Besonders vorteilhaft ist es, wenn das Material homogen vorliegt bzw. im Wesentlichen homogen, wobei unter einer homogenen Ausgestaltung des Materials vorzugsweise bestanden wird, dass das Material als einphasige Keramik (oder Optokeramik) vorliegt.

Gemäß einer weiteren Ausführungsform weist der Konverter mindestens zwei keramische Konvertermaterialien auf, welche das Laserlicht in Licht unterschiedlicher spektraler Zusammensetzungen konvertieren. Eine solche Ausgestaltung kann insbesondere dann vorteilhaft sein, wenn besonders genaue und/oder detailreiche Untersuchungen zur Bestimmung des Zustands des untersuchten Gegenstands oder Bereichs erforderlich sind, insbesondere im medizinischen Sektor, wenn genaue Informationen über den Zustand des untersuchten Gewebes notwendig sind, beispielsweise um Behandlungs- und/oder Therapiepläne zielgerichtet erstellen zu können. Denn auf diese Weise ist es möglich, eine hohe Beleuchtungsintensität zu erzielen und Licht einer beispielsweise von einem "weißen" Farbort abweichenden Zusammensetzung zu erhalten und/oder die spektrale Zusammensetzung des Lichts fallweise anzupassen.

Besonders vorteilhaft umfasst der Konverter zwei Konverterelemente, wobei die Konverterelemente jeweils eines der keramischen Konvertermaterialien enthalten, sodass die Konverterelemente das Licht in Licht unterschiedlicher spektraler Zusammensetzung konvertieren. Auf diese Weise gelingt nämlich eine besonders leichte Anpassung des Farborts, insbesondere ist es bei einer solchen Ausgestaltung besonders einfach möglich, durch eine entsprechende Steuerung nur eines der beiden Konvertermaterialien zu beleuchten und/oder das Laserlicht auf die beiden Konvertermaterialien entsprechend aufzuteilen.

Gemäß einer Ausführungsform ist der Konverter optisch so mit dem Lichtleiter gekoppelt, dass vom Konverter remittiertes Licht in den Lichtleiter eingekoppelt ist oder wird oder zumindest einkoppelbar ist. Dies ist vorteilhaft, um sicherzustellen, dass gerade Licht mit gewünschter Spektralverteilung durch den Lichtleiter auf den mittels des Endoskops zu untersuchenden Bereich geleitet wird. Unter remittiertem Licht wird im Rahmen der vorliegenden Offenbarung, sofern nicht ausdrücklich anders ausgeführt, Licht verstanden, welches vom Konverter konvertiert und/oder gestreut und/oder reflektiert wird.

Gemäß einer weiteren bevorzugten Ausführungsform ist der Laser so angeordnet und auf den Konverter gerichtet, dass ausschließlich vom Konverter konvertiertes und/oder gestreutes und/oder reflektiertes Licht in den Lichtleiter eingekoppelt wird, wobei dieses Licht auch Anteile von z.B. gestreutem oder reflektiertem Primärlicht umfassen kann.

Eine solche Ausgestaltung des Endoskops ist insbesondere unter Sicherheitsgesichtspunkten vorteilhaft, da auf diese Weise verhindert wird, dass Laserlicht in den zu untersuchenden Bereich gelangt.

Es ist allgemein möglich, dass der Konverter und der Laser in einem sogenannten Transmissionsaufbau angeordnet sind, also dass das Licht des Lasers durch den Konverter hindurch geht, also transmittiert, und dabei konvertiert und/oder gestreut wird. Es ist aber auch möglich und kann sogar bevorzugt sein, insbesondere um sicherzustellen, dass kein Laserlicht, also unkonvertiertes und/oder ungestreutes vom Laser ausgesendetes Licht, an den zu untersuchenden Bereich gelangt, dass der Konverter und der Laser in Reflexion angeordnet sind, also das Laserlicht auf den Konverter fällt und von diesem reflektiert und dabei konvertiert und/oder gestreut wird.

Gemäß einer weiteren Ausführungsform ist der Laser so angeordnet, dass das Licht des Lasers im Wesentlichen entgegen der Lichtabstrahlrichtung des vom Konverter konvertierten und/oder gestreuten und/oder reflektierten und in den Lichtleiter eingekoppelten Lichts gerichtet wird und/oder gerichtet ist und/oder auf diesen lenkbar ist. Gerade eine solche Ausgestaltung kann unter Sicherheitsaspekten besonders vorteilhaft sein, um zu verhindern, dass direktes Laserlicht in den Lichtleiter eingekoppelt wird. Eine solche Ausgestaltung des Endoskops kann beispielsweise dadurch ermöglicht werden, dass das Endoskop ein Mittel aufweist, mittels dessen das Licht des Lasers wie ausgeführt entgegen der Lichtabstrahlrichtung des remittierten Lichts aus den Konverter gerichtet wird. Beispielsweise kann das Mittel eine lichtleitende Faser sein und/oder eine solche umfassen.

Unter einer Einstrahlrichtung im Wesentlichen entgegen der Lichtabstrahlrichtung des vom Konverter konvertierten und in den Lichtleiter eingekoppelten Lichts wird hierbei verstanden, dass zwischen dem Normalenvektor auf die Oberfläche des Konverters und/oder des Konverterelements und/oder der Konverterelemente und der Einkopplungsrichtung des Primärlichtes ein Winkel von mindestens ± 10° gebildet ist.

Geeignete Lichtleiter können beispielsweise einige Zehn, einige Hundert bis hin zu einigen Tausend Einzelfasern für derartige Endoskopsysteme umfassen, wobei die genaue Zahl der Einzelfasern, welche vom Lichtleiter umfasst sind, beispielsweise abhängig ist vom adressierten Enddurchmesser des Lichtleiters und/oder dem Durchmesser vom Lichtleiter umfassten Einzelfasern. Die normal üblichen Faser-Durchmesser betragen zwischen 20 µm und 100 µm. Typisch sind Durchmesser von 30 µm, 50 µm und 70 µm.

Insbesondere für Single-Use-Endoskope bzw. für Endoskopsysteme mit kleinen Abmessungen kann vorteilhaft sein, wenige dicke Fasern als lichtleitende Fasern zu verwenden, um sicherzustellen, dass eine ausreichende Leuchtdichte bzw. Beleuchtungsintensität am zu untersuchenden Bereich gewährleistet ist. Einerseits kann damit eine kostengünstige schnelle Montage erfolgen und anderseits ein hoher Lichtstrom von der Laser-Lichtquelle kommend zum distalen Ende des Endoskops gewährleistet werden.

Als vorteilhaft und als guter Kompromiss zwischen Montageaufwand und ausreichender Lichtstromübertragung hat sich eine Anzahl von höchstens zwanzig, vorzugsweise höchstens zehn, derartiger Einzelfasern herausgestellt, wobei für extrem dünne Endoskopsysteme bereits eine Faser ausreichend sein kann. Bündel aus drei oder sieben Einzelfasern bieten den Vorteil, dass diese sehr dicht in einer gemeinsamen Hülse gepackt werden können. Die 7er-Anordnung bildet dabei den besonderen Vorteil, dass sich in der gemeinsamen Hülse eine eher kreisrunde Anordnung der Einzelfasern verwirklichen lässt und zudem eine für Fasern mit rundem Querschnitt ideale Packungsdichte ergibt. Bei einer solchen 7er-Anordnung können dann beispielsweise am distalen Ende des Endoskops die Einzelfasern um den Kamerachip oder um die Bildleiter derart gruppiert werden, dass eine gleichmäßige Ausleuchtung des zu untersuchenden Gewebes ermöglicht werden kann. Im Hinblick auf die eher übliche quadratische Chip-Form von Laser- oder Leuchtdioden oder des Konverters als Lichtquelle kann es aber auch vorteilhaft sein, wenn vier Enzelfasern oder ganzzahlige Vielfache von vier oder auch zwei derartiger Fasern verwendet werden. Einerseits können die für die Beleuchtung zur Verfügung stehenden Kavitäten hinsichtlich einer möglichst hohen aktiven Faserfläche, also der eigentlich lichtleitende Querschnittsbereich der Faser, mit mehr Fasern gefüllt werden, und anderseits eine bessere Lichteinkopplung erzielt werden.

Hierbei hat sich als vorteilhaft erwiesen, dass die eine lichtleitende Faser oder die mehreren lichtleitenden Fasern einen Durchmesser im Bereich von 100 µm bis 1000 µm, bevorzugt bis 600 µm, vorzugsweise im Bereich von 150 µm bis 400 µm, aufweisen. Derartige Fasern lassen sich als Einzelfaser deutlich einfacher montieren und weisen noch einen ausreichend kleinen minimalen Biegeradius auf. Für heutige Endoskope mit beispielsweise einem Kamera-Chip der Grüße 1 × 1 mm² wären beispielsweise vier Enzelfasern, eine an jeder Seite der Kamera angeordnet, mit einem Durchmesser im Bereich von 200 µm bis 300 µm ideal. Ebenso bevorzugt sind Anordnungen mit in Summe acht oder zwölf Einzelfasern, je zwei oder drei Fasern an jeder Seite der Kamera angeordnet, wobei in diesem Fall die Einzelfasern einen Durchmesser im Bereich von 150 µm bis höchstens 200 µm aufweisen. Es kann auch vorgesehen sein, dass beispielsweise Fasern mit unterschiedlichem Durchmesser verwendet werden, um eine möglichst gute Ausfüllung der Fläche bzw. des zu Verfügung stehenden Platzes zwischen Kamera-Chip und umgebender Hülle zu ermöglichen, so dass ein möglichst hoher Lichtstrom erzielt werden kann. Bei einer 12er-Faseranordnung, drei Fasern pro Kamera-Seite, könnte beispielsweise die mittlere Faser einen Durchmesser von etwa 250 µm aufweisen, wobei die beiden anderen Fasern nur einen Durchmesser von 100 µm bis 150 µm aufweisen.

Grundsätzlich können statt der Einzelfasern auch dünne Faserbündel Verwendung finden, welche insbesondere aus sehr dünnen Einzelfasern mit einem Einzelfaserdurchmesser von bevorzugt kleiner 70 µm, besonders bevorzugt kleiner 50 µm bestehen, üblicherweise 30 µm, welche nur einen extrem dünnen Mantel aufweisen, welcher das Faserbündel zusammenhält. Derartige Faserbündelausführungen sind in einer noch nicht veröffentlichten Parallel-Anmeldung der Anmelderin beschrieben.

Gemäß einer weiteren Ausführungsform ist die eine lichtleitende Faser oder sind die mehreren lichtleitenden Fasern Stufenindex-Glasfasern. Vorzugsweise ist die eine lichtleitende Faser oder sind die mehreren lichtleitenden Fasern Stufenindex-Glasfasern umfassend eine Glaszusammensetzung, die bis auf unvermeidliche Spuren frei ist von Blei und/oder sonstigen Schwermetallen sowie frei von Antimon und/oder Arsen und/oder von sonstigen kritischen Elementen wie Cr(VI) ist.

Unter einer Faser wird im Rahmen der vorliegenden Offenbarung ein Körper verstanden, dessen größte laterale Abmessung in einer Raumrichtung eines kartesischen Koordinatensystems mindestens um den Faktor 10, vorzugsweise mindestens um den Faktor 50, größer ist als in den beiden anderen, zu dieser ersten Raumrichtung senkrechten Raumrichtungen. Mit anderen Worten ist eine Faser ein sehr langer, dünner Körper.

Unter einer Stufenindex-Glasfaser wird im Rahmen der vorliegenden Offenbarung eine Glasfaser verstanden, deren Brechungsindex sich von innen, dem Kern, nach außen, in Form mindestens einer Stufe ändert. Dabei umfasst die Glasfaser ein Kernglas und ein Mantelglas, wobei das Kernglas einen anderen Brechungsindex aufweist als das Mantelglas.

Eine Glasfaser umfasst Glas. Die Glasfaser kann weiterhin neben dem glasigen Material noch ein weiteres Material umfassen, welches die Oberfläche des glasigen Materials zumindest teilweise umgibt, die sogenannte Schlichte. Je nach Einsatzzweck können unterschiedliche glasige Materialien für eine Glasfaser verwendet werden. Insbesondere kann die Glasfaser ein einkomponentiges und/oder ein mehrkomponentiges Glas umfassen. Beispielsweise kann die Glasfaser als im Wesentlichen einkomponentiges Glas Quarzglas umfassen und/oder insbesondere als Quarzglasfaser ausgebildet sein, wobei das Quarzglas auch dotiert vorliegen kann, beispielsweise mit OH-Ionen und/oder mit Fluor dotiert, und/oder als bspw. wasserreiche oder wasserarme Quarzglasvarianten vorliegt, wobei in diesem Fall noch immer von einem einkomponentigen Glas gesprochen wird, oder ein mehrkomponentiges Glas umfassen, beispielsweise ein mehrkomponentiges, silikatisches Glas. Weiterhin kann das Glas auch als Chalkogenidglas ausgebildet sein. Unter einer Quarzglasfaser oder Quarzfaser wird dabei auch eine Faser umfassend dotiertes Quarzglas verstanden.

Bevorzugt umfasst die optische Faser einen Faserkern und einen Faserrand bzw. eine Fasermantelschicht. In bevorzugten Ausführungsformen besteht die Kernschicht aus einem Kernglas.

Bevorzugt umfasst die optische Faser einen Fasermantel, welche den Faserkern umgibt. In bevorzugten Ausführungsformen umfasst der Fasermantel ein Mantelglas.

Bevorzugt weist der Fasermantel einen Gehalt an Halogenen bzw. Halogeniden von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist der Fasermantel frei von Halogen. Halogene sind beispielsweise Chlor, Fluor, Brom und/oder lod bzw. deren Anionen. Eine zu hohe Konzentration an Halogenen Im Fasermantel führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

Bevorzugt weist der Faserkern einen Gehalt an Halogenen bzw. Halogeniden von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist die Kernschicht frei von Halogen.

Beispielhafte Halogene sind Chlor, Fluor, Brom und/oder lod bzw. deren Anionen. Eine zu hohe Konzentration an Halogenen im Faserkern führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

In bestimmten Ausführungsformen ist die optische Faser eine Quarzfaser. In einer bestimmten Ausführungsform weist der Fasermantel und/oder weist der Faserkern einen Anteil von mindestens 76 Gew.-% Quarz auf, weiter bevorzugt mindestens 81 Gew.-%, weiter bevorzugt mindestens 84 Gew.-%, weiter bevorzugt mindestens 88 Gew.-%, weiter bevorzugt mindestens 92 Gew.-%, weiter bevorzugt mindestens 95 Gew.-%, weiter bevorzugt mindestens 97 Gew.-%, weiter bevorzugt mindestens 98 Gew.-%. Ein höherer Quarzanteil führt zu einer erhöhten chemischen Beständigkeit sowie zu einer erhöhten Temperaturbeständigkeit.

In einer bestimmten Ausführungsform weist das Kernglas folgende Merkmale auf:
Bevorzugt umfasst das Kernglas wenigstens 8 Gew.-%, weiter bevorzugt wenigstens 23 Gew.-%, mehr bevorzugt wenigstens 24 Gew.-% und besonders bevorzugt wenigstens 25 Gew.-% oder sogar wenigstens 26 Gew.-% SiO₂. In einer besonderen Ausführungsform kann das Kernglas sogar mindestens 28,3 Gew.-% SiO₂ umfassen, ganz besonders bevorzugt mindestens 34 Gew.-% SiO₂. In einigen bevorzugten Ausführungsformen umfasst das Kernglas sogar wenigstens 35 Gew.-% SiO₂, weiter bevorzugt wenigstens 42 Gew.-%.

Bevorzugte Kerngläser dieser Erfindungen umfassen die folgenden Komponenten in den folgenden Zusammensetzungsbereichen in Gewichtsprozent:

| **Komponente** | **von** | **bis** |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

R₂O ist jeweils die Summe der Gehalte aller Alkalimetalloxide.

Eine oder mehrere der folgenden Komponenten kann vom Kernglas umfasst sein: Cs₂O, Rb₂O, MgO, CaO, SrO, Gd₂O₃, Lu₂O₃, Sc₂O₃, Y₂O₃, In₂O₃, Ga₂O₃ und WO₃.

Folgende Komponenten sollten im Kernglas bevorzugt nicht enthalten sein oder lediglich in Konzentrationen von jeweils höchstens 500 ppm, die durch unvermeidbare Verunreinigungen der Rohstoffe bedingt sind: TiO₂, CeO₂, Nb₂O₅, MoO₃, Bi₂O₃, PbO, CdO, Tl₂O, As₂O₃, Sb₂O₃, SO₃, SeO₂, TeO₂, BeO, radioaktive Elemente und färbende Komponenten, soweit im Text nicht anders beschrieben. Insbesondere auf TiO₂ sollte verzichtet werden, weil diese Komponente für eine ausgeprägte Absorption im UV-Bereich führen kann. In bevorzugten Ausführungsformen wird auch auf die Komponente WO₃ verzichtet.

Die Komponenten TiO₂, CeO₂, Nb₂O₅ und/oder Bi₂O₃ können bis maximal 0,5 Gew.-%, bevorzugt bis 0,3 Gew.-% und besonders bevorzugt bis 0,2 Gew.-% im Kernglas enthalten sein. In einer bevorzugte Ausführungsform ist das Kernglas frei von diesen Komponenten.

Bevorzugt ist das Kernglas frei von optisch aktiven Komponenten, insbesondere Sm₂O₃, Nd₂O₃, Dy₂O₃, Pr₂O₃, Eu₂O₃, Yb₂O₃, Tb₂O₃, Er₂O₃, Tm₂O₃ und/oder Ho₂O₃. CeO₂ absorbiert im UV-Bereich, so dass bevorzugte Kerngläser kein CeO₂ enthalten.

Der Gehalt der Komponenten Erdalkalimetalloxide, La₂O₃, Ta₂O₅, ZrO₂ und HfO₂ beträgt in Summe vorzugsweise und insbesondere für Kerngläser mit Brechwerten von mehr als 1,65 wenigstens 40 Gew.-%, weiter bevorzugt wenigstens 42 Gew.-%, mehr bevorzugt wenigstens 50 Gew.-% und besonders bevorzugt wenigstens 55 Gew.-%. Wenn der Gehalt dieser Komponenten zu niedrig ist, kann der bevorzugte Brechungsindex normalerweise nicht erreicht werden. Formulierungsbedingt sollte diese Summe einen Wert von 72 Gew.-% nicht übersteigen.

In einer bestimmten Ausführungsform weist das Mantelglas folgende Merkmale auf:
Bevorzugt weist das Mantelglas einen Gehalt an SiO₂ von >60 Gew.-%, weiter bevorzugt >65 Gew.-% und besonders bevorzugt von mindestens 69 Gew.-% auf. Der SiO₂-Gehalt beträgt bevorzugt höchstens 75 Gew.-% und besonders bevorzugt bis zu 73 Gew.-%. Das Mantelglas ist tendenziell stärkeren Umwelteinflüssen ausgesetzt als das Kernglas. Ein hoher SiO₂-Gehalt verleiht bessere chemische Resistenz. Folglich ist der Gehalt an dieser Komponente im Mantelglas bevorzugt größer als im Kernglas.

Vorzugsweise wird die Zusammensetzung des Mantelglases so ausgewählt bzw. an die des Kernglases angepasst, dass der lineare thermische Ausdehnungskoeffizient des Mantelglases und derjenige des Kernglases sich möglichst wenig unterscheiden. Allgemein kann der thermische Ausdehnungskoeffizient (CTE) in einem Temperaturbereich von 20 bis 300°C für Faserkern und Fasermantel gleich oder unterschiedlich sein. Insbesondere ist der CTE unterschiedlich. Vorzugsweise ist der CTE des Mantels kleiner als der CTE des Faserkerns, typischerweise ist er mindestens um 1,0*10⁻⁶ /K kleiner, kann aber auch, je nach Glas typischerweise mindestens um 2,5*10⁻⁶ /K. kleiner sein. Der Faserkern weist typischerweise einen CTE von 6,5*10⁻⁶ bis 10*10⁻⁶/K auf, der Mantel einen CTE von 4,5*10⁻⁶ bis 6*10⁻⁶/K. Dadurch wird erreicht, dass der Kern der Faser beim Abkühlen stärker als der Fasermantel schrumpft, wodurch im Fasermantel eine die Faser schützende Druckspannung aufgebaut wird, was für die mechanische Belastbarkeit der Faser, insbesondere ihrer Biegefestigkeit zuträglich ist.

Die folgende Tabelle zeigt einige bevorzugte Zusammensetzungen von Mantelgläsern, die zusammen mit den Kerngläsern verwendet werden können. Die Mantelgläser umfassen (in Gew.-% auf Oxidbasis):

| **Oxide** | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5-10 | 1 -7 | 1-5 | 1 - 10 |
| B₂O₃ | 5-14 | 0-3 | 10-14 | > 15 |
| Li₂O | frei | 0-1 | 0-3 | < 0,1 |
| Na₂O | 0-10 | 8-20 | 2-8 | 0-10 |
| K₂O | 0-10 | 0-6 | 0-1 | 0-10 |
| MgO | 0-1 | 0-5 | frei | 0-5 |
| CaO | 0-2 | 1 - 9 | frei | 0-5 |
| SrO | 0-1 | frei | frei | 0-5 |
| BaO | 0-1 | 0-5 | frei | 0-5 |
| Halogen | frei | frei | frei | frei |

In einer anderen bestimmten Ausführungsform ist das Kernglas und/oder das Mantelglas ein Chalcogenidglas, welches insbesondere Anwendungen im Infrarot-Bereich ermöglicht. Die folgende Tabelle zeigt bevorzugte Zusammensetzungen von Kernchalcogenidgläsern und/oder Mantelchalcogenidgläsern in Molprozent:

| **Komponente** | **Mol-%** |
|---|---|
| S | 50-90 |
| Ga | 0-25 |
| As | 0-40 |
| Ge | 0-35 |
| R¹ (zugegeben in Form von R¹Hal) | 0-7,25 |
| R² (zugegeben in Form von R²Hal) | 0-13,5 |
| M¹ (zugegeben in Form von M¹Hal₂) | 0-5 |
| M² (zugegeben in Form von M²Hal₂) | 0-7,25 |
| Ln (zugegeben in Form von LnHal₃) | 0-4 |
| Sum of Ga, As, and Ge | 10-42 |
| Sum of R¹, R², M¹, M², and Ln | 0-16 |
| Sum of Hal | 0-16 |

| | |
|---|---|
| Hierbei ist Hal = Fluor, Chlor, Brom, und/oder lod; Hal₂ und/oder Hal₃ = Chlor und/oder Brom; R¹ = Li, Na, K, Rb, und/oder Cs; R² = Ag und/oder Cu; M¹ = Mg, Ca, Sr, und/oder Ba; M² = Zn, Cd, Hg, und/oder Pb; Ln = La, Ce, Pr, Nd, Pm, Sm Eu, Gd, Tb, Dy, Ho, Er, Tm, Ty, Lu, Y, und Sc. | |

Besonders vorteilhaft ist es, wenn die Glasfasern, Faserstäbe oder gepressten Faserstäbe aus einem Pb- bzw. Schwermetall-freien Kernglas und Mantelglas bestehen. Derartige Fasersysteme bieten insbesondere eine hohe Transmission im VIS-Spektralbereich und zeigen aufgrund der vergleichsweisen hohen Transmission im blauen Spektralbereich eine hohe Farbtreue, was insbesondere wichtig ist bei der medizinischen Beurteilung von Gewebe. Oft entscheiden hier nur geringfügige Farbunterschiede des Gewebes, ob es sich um eine gutartige oder bösartige Gewebeveränderung handelt. Daher kommt es auf einen hohen CRI-Wert des Gesamtsystems aus Lichtquelle, Lichtleiter und bildgebender Einrichtung an, wobei CRI (Color Rendering Index) eine Kennzahl einer photometrischen Größe ist, mit der die Qualität der Farbwiedergabe von Lichtquellen gleicher korrelierter Farbtemperatur beschrieben wird. Ein CRI-Wert von > 90 kann mit den oben beschriebenen Glasfasern, Faserstäbe oder gepressten Faserstäbe erzielt werden. Derartige Fasersysteme sind seitens der Anmelderin unter dem Namen SCHOTT PURAVIS^{®} bekannt und sind hinsichtlich ihrer Zusammensetzungen in der DE 102012100233 B4 und DE 102013208838 B4 beschrieben. Ähnliche Fasersysteme sind auch in der EP 2072477 B1 beschrieben, welche ebenfalls Pb-frei sind.

Insbesondere für die Verwendung in Endoskopen ist es von Vorteil, wenn Glasfasern, Faserstäbe oder gepressten Faserstäbe aus einem Glassystem bestehen, welches für das zu leitende Licht einen Akzeptanzwinkel 2a von größer 80°, besonders bevorzugt von größer 100° aufweist, was einer numerischen Apertur (NA) von größer 0,64, besonders bevorzugt größer 0,77 entspricht. Zum einen kann erreicht werden, dass insbesondere Licht von LEDs, welche i.d.R. einen sehr breiten Abstrahlwinkel aufweisen, ohne komplexe Optiken am proximalen Ende in die Glasfasern bzw. Faserstäbe oder gepressten Faserstäbe einkoppelbar sind, ohne dass erhöhte Einkoppelverluste entstehen. Andererseits kann am distalen eine weitwinklige Ausleuchtung ohne zusätzlich erforderliche Optik erreicht werden, was insbesondere endoskopische Untersuchungen bevorzugt. Eine optimale Ausleuchtung bei den derzeit üblichen Kamera-Sichtwinkeln (üblicherweise 120° diagonal) kann dann erzielt werden, wenn die Glasfasern, Faserstäbe oder gepressten Faserstäbe einen Akzeptanzwinkel 2α von mindestens 120° bzw. eine NA von mindestens 0,86 aufweisen.

Glasfasern, wie sie zuvor beschrieben worden, weisen nach ihrem Ziehprozess normalerweise eine weitgehend unverletzte, feuerpolierte Oberfläche auf, die es gilt möglichst vor Verletzungen zu schützen. Dazu werden bei Glasfasern sogenannte Schlichten vor dem Wickel-Prozess aufgebracht, die die Fasern insbesondere beim gegenseitigen Reiben der Fasern untereinander aber auch bei Kontakt mit z.B. Metalloberflächen schützen. Derartige Schlichten bestehen i.d.R. aus Wachs- bzw. Stearin-basierten Lösungen, die auf die Glasfasern aufgesprüht werden. In einer noch nicht veröffentlichten Anmeldung der Anmelderin werden weitere dieser Schlichten beschrieben.

Hinsichtlich einer weiteren mechanischen Stabilisierung der Faser, insbesondere bei Fasern mit größerem Durchmesser, wie zuvor beschrieben, hat sich als vorteilhaft herausgestellt, wenn die eine oder die mehreren lichtleitenden Fasern eine zumindest teil- und/oder abschnittsweise an deren Mantelfläche angeordnete polymerbasierte Beschichtung oder eine Schutzhülle aus einem polymerbasiertem Schlauchmaterial, z.B. als Schrumpfschlauch ausgeführt, aufweisen. Dabei kann eine höhere Festigkeit und damit auch kleinere Biegeradien der Fasern erzielt werden. Der an sich resultierende Nachteil einer dickeren Faser hinsichtlich zunehmender Steifigkeit und Zunahme des minimal zulässigen Biegeradius kann mit dieser Maßnahme deutlich abgeschwächt oder kompensiert werden.

Gemäß einer Ausführungsform umfasst der Lichtleiter mehrere lichtleitende Fasern, wobei wenigstens eine lichtleitende Faser, bevorzugt mehrere lichtleitende Fasern, besonders bevorzugt alle lichtleitenden Fasern, eine zumindest teil- und/oder abschnittsweise an deren Mantelfläche angeordnete polymerbasierte Beschichtung oder eine Schutzhülle aus einem polymerbasiertem Schlauchmaterial aufweist.

Vorteilhaft sind Beschichtungen aus einer Acrylat-, Polyamid-, Polyurethan-, Polyimid-, Epoxi-, Ethylen-Tetrafluorethylen-Copolymer- oder Poly-Xylol-basierten Verbindungen (auch als Poly-Xylylen-basierte Beschichtungen bezeichnet), beispielsweise basierend auf poly-para-Xylol-Verbindungen, beispielsweise auch unter dem Handelsnamen "Parylene" als Beschichtungsmaterial bekannt, oder Mischungen dieser Verbindungen. Geeignete Beschichtungsmaterialien sind beispielsweise unter den Handelsnamen oder Marken oder Bezeichnungen NYLON^{®} (Polyamid) oder TEFZEL^{®} oder Parylene^{®} oder PMMA (Polymethylmethacrylat) als Coatings oder Beschichtungen oder Beschichtungsmaterialien erhältlich. Üblicherweise erfolgt bei diesen Schichten eine Aushärtung durch Aufheizen oder mittels UV-Licht. Alternativ oder zusätzlich kann die Beschichtung auch umfassen thermoplastische Elastomere, beispielsweise ein thermoplastisches Polyesterelastomer oder ein thermoplastisches Copolyesterelastomer, wie es beispielsweise unter dem Handelsnamen Hytrel kommerziell erhältlich ist, oder ein Silikon.

In speziellen Fällen können auch metallische Beschichtungen, beispielsweise aus Gold oder Aluminium zum Einsatz kommen.

Besonders vorteilhaft ist es, wenn eine derartige Beschichtung mittels Tauchen, Besprühen, Extrudieren oder Abscheidung bei niedrigem Druck auf die eine oder die mehreren lichtleitenden Fasern unmittelbar nach dem Ziehen der Fasern aufbringbar ist oder aufgebracht ist. Insbesondere kann eine solche Beschichtung mittels Tauchen, Besprühen, Extrudieren oder Abscheidung bei niedrigem Druck auf die eine oder die mehreren lichtleitenden Fasern, beispielsweise unmittelbar nach dem Ziehen der Fasern, aufgebracht werden. Insbesondere mit einem Aufbringen unmittelbar nach dem Ziehen der Faser oder der Fasern kann erreicht werden, dass die nahezu perfekte, feuerpolierte Oberfläche der Faser oder der Fasern konserviert werden kann, bevor sie in Kontakt mit anderen Materialien oder mit anderen Fasern kommt. Damit können Mikro-Beschädigungen, die die Festigkeit der Faser oder der Fasern reduzieren, zumindest vermindert werden. Auch ist es prinzipiell sogar denkbar, dass eine solche Beschichtung auch zu einem Ausheilen von etwaigen Vorschädigungen führen kann bzw. die Wirkung solcher Vorschädigungen zumindest teilweise reduziert. Auch kann ein Schutz vor hydrolytischem Angriff erzielt werden.

Üblicherweise werden derartige Schichten derart aufgebracht, dass der frisch gezogenen Lichtleiter als Faser durch einen Topf mit einer Düse gezogen wird, in dem sich das zu Beschichtung dienende Polymermaterial befindet, wobei mit der Düse u.a. auch die Schichtdicke eingestellt werden kann.

Die Schichtdicke dieser Beschichtung liegt typischerweise im Bereich von 5 µm bis 100 µm, vorzugsweise im Bereich von 10 µm bis 50 µm.

Zudem kann ergänzend vorgesehen sein, dass zusätzlich zu dieser ersten Beschichtung noch mindestens eine weitere organische Beschichtung aufgebracht werden kann. Derartige zusätzliche Beschichtungen werden auch als Buffer bezeichnet und finden üblicherweise bei Quarzfasern Anwendung. Als Materialien für derartige Buffer kommen beispielsweise PMMA, Polyamid (NYLON^{®}), Polyimid oder fluorierte Polymere, wie ein Ethylen-Tetrafluorethylen-Copolymer (Kurzzeichen ETFE), welches beispielsweise unter dem Handelsnamen TEFZEL^{®} kommerziell erhältlich ist, in Betracht. Eine derartig weitere Beschichtung dient zur Steigerung der Robustheit hinsichtlich der Biegebelastbarkeit. Insbesondere kann diese Bufferschicht auch umfassen ein thermoplastisches Elastomer, beispielsweise ein thermoplastisches Polyesterelastomer oder ein thermoplastisches Copolyesterelastomer, wie es beispielsweise unter dem Handelsnamen Hytrel^{®} kommerziell erhältlich ist, und/oder Polyvinylidenfluorid, beispielsweise erhältlich unter dem Handelsnamen Kynar, oder Polytetrafluoroethylen (beispielsweise erhältlich unter dem Handelsnamen Teflon) oder auch Polyurethan. Solche Buffer-Beschichtungen können beispielsweise aufgebracht werden durch Sprühen, Tauchen, Extrusion und elektrostatische Methoden.

Ein derartiges Schichtsystem kann beispielsweise aus einem zweilagigen System bestehen, bei dem zunächst eine vergleichsweise dünne Schicht, typischerweise 10 µm bis 50 µm dick, z.B. aus einer Acrylat- oder Epoxyverbindung, auf den Lichtleiter appliziert wird, und anschließend nochmals als weiterer mechanischer Schutz eine sogenannte Buffer-Schicht beispielsweise aus NYLON^{®}, TEFZEL^{®}, PMMA oder Polyimid aufgebracht wird, die dann auch eine deutlich größere Wandstärke, typischerweise 50 µm bis 200 µm, aufweisen kann.

Für Anwendungen mit sehr geringen Platzverhältnissen genügt bereits das erste Coating, um eine möglichst hohe Biegefestigkeit zu gewährleisten.

Hier sei angemerkt, dass auch andere Verfahren denkbar sind, um insbesondere die Festigkeit der Faser zu erhöhen. So könnte beispielsweise durch gezielte Temperatur-Prozesse ähnlich dem thermischen Vorspannen von Glas oberflächennah eine höhere Druckvorspannung aufgebaut werden, was die Biegefestigkeit der Faser erhöhen kann. Denkbar ist auch das chemische Härten der Faser. Hierzu wäre allerdings, um die optischen Eigenschaften der Faser zu erhalten, ein weiterer Mantel notwendig, in dem durch Ionen-Austausch in einer Salzschmelze oder durch Aufsprühen einer Salz-Schicht mit nachfolgender Temperung eine gezielte zusätzliche Druckvorspannung in diesem zusätzlichen Mantel aufgebaut werden kann. Ebenso denkbar wäre ein Elektronen- oder Ionenstrahl-Härten. Allerdings sind diese letztgenannten Verfahren vergleichsweise aufwendig. Zudem ist es damit schwierig, die optischen Eigenschaften der Fasern beizubehalten.

Die Beschichtung kann gemäß einer weiteren Ausführungsform auch lichtblockend ausgeführt sein, also opak oder lichtabsorbierend, beispielsweise farbig, wie schwarz oder blau, ausgeführt sein. Dies ist vorteilhaft, weil auf diese Weise ein Übersprechen auf den Kamerachip reduziert werden kann.

Eine Ausführungsform, bei welchem der Lichtleiter mindestens eine Glasfaser, insbesondere eine Glasfaser umfassend ein mehrkomponentiges silikatisches Glas oder eine Glasfaser aus einem mehrkomponentigen silikatischen Glas, umfasst oder bevorzugt als Glasfaserbündel, insbesondere als Glasfaserbündel umfassend Glasfasern umfassend ein mehrkomponentiges, silikatisches Glas oder aus einem mehrkomponentigen silikatischen Glas oder aus Glasfasern aus einem mehrkomponentigen silikatischen Glas, ausgebildet ist, ist dabei besonders vorteilhaft.

Denn mit solchen Glasfasern sind die optischen Eigenschaften des diese Glasfasern umfassenden Glasfaserbündels und mithin des Lichtleiters bzw. des Endoskops besonders flexibel einstellbar. Weiterhin weisen solche Lichtleiter, die auf Glasfasern, basieren, eine deutlich höhere Temperaturbeständigkeit als eine polymere optische Faser (POF) auf. Dies ist insbesondere dann relevant, wenn eine besonders gute Einkoppeleffizienz realisiert werden soll, beispielsweise dann ein dünnes Faserbündel aus oder umfassend Glasfasern direkt auf einen LED-Chip kontaktiert werden oder sehr nahe an einen solchen Chip herangeführt werden. Eine polymere optische Faser bzw. ein Faserbündel aus oder umfassend polymere optische Fasern würde einer solchen thermischen Belastung jedoch nicht standhalten, sondern es würde zum Schmelzen der Fasern kommen.

Gemäß einer Ausführungsform ist die eine lichtleitende Faser oder sind die mehreren lichtleitenden Fasern am proximalen Ende in eine Einkoppelhülse gefasst, welche als mechanisches Interface zur Laser-Lichtquelle ausgeführt ist und damit eine definierte Lichteinkopplung hinsichtlich Fokusabstand und Zentrierung zur Lichtquelle ermöglicht. Bei einer Einzelfaser oder bei mehreren Einzelfasern, idealerweise drei oder sieben Einzelfasern, können beispielsweise sogenannte SMA-Stecker als Einkoppelhülse vorgesehen sein, die insbesondere eine definierte Ausrichtung zur Laser-Lichtquelle ermöglichen und auch insbesondere für Laser-Anwendungen gebräuchlich sind. Ebenso denkbar sind dafür auch sogenannte FC-Stecker. Hier ist insbesondere wieder eine Anordnung aus sieben Einzelfasern besonders vorteilhaft, da hier im Wesentlichen ein kreisrunder Querschnitt zu einen und zum anderen eine minimierte Zwickelfläche zwischen den Einzelfasern ermöglicht wird. Dies hat Vorteile hinsichtlich der Einkoppeleffizienz. Unter Zwickel versteht man die Zwischenräume bei einem Bündel aus kreisrunden Fasern. Eine weitere optimale Faseranordnung ergäbe sich bei 19 Einzelfasern, bei denen dann die Einzelfasern optimal dicht gepackt in zwei Schalen um eine Zentralfaser angeordnet sind. Die Fixierung der Einzelfasern erfolgt dabei i.d.R. mittels eines Klebers, z.B. eines zweikomponentigen heißvernetzenden Epoxy-Klebers oder mittels eines UV-aushärtenden Klebers.

Um die Einkoppeleffizienz zu steigern, kann zudem vorgesehen sein, dass die lichtleitenden Fasern am proximalen Ende heiß-verschmolzen angeordnet sind. Hierbei können Zwickelflächen zum einen minimierten werden, da durch den Heiß-Verformungsprozess die an sich runden Einzelfasern zu einer zumindest annähernden hexagonalen Querschnittsfläche verformt werden und sich damit nahezu lückenlos anordnen lassen. Zudem können bei vorgegebenen Einkoppelquerschnitt bzw. Fokusdurchmesser mehr Fasern untergebracht und damit ein höherer Lichtstrom übertragen werden.

Es ist möglich, dass solche heißverschmolzenen Fasern beispielsweise am proximalen Ende in einer Einkoppelhülse angeordnet sind. Es ist jedoch ebenfalls möglich, dass die heißverschmolzenen Fasern hülsenlos am proximalen Ende vorliegen. Dies ist insbesondere für Ausgestaltungen vorteilhaft, bei welchen eine effiziente Raumnutzung erforderlich ist, also beispielsweise bei besonders geringen Flächenquerschnitten am proximalen Ende o.ä.

Gemäß einer weiteren Ausführungsform ist die zumindest eine lichtleitende Faser und/oder sind die mehreren lichtleitenden Fasern und oder ist der Lichtleiter am distalen Ende gegenüber dem proximalen Ende verformt. Dies bedeutet, dass die zumindest eine lichtleitende Faser und/oder dass die mehreren lichtleitenden Fasern und/oder dass sogar der Lichtleiter selbst gemäß einer Ausführungsform eine Querschnittsfläche aufweisen kann, welche am distalen Ende eine andere Form aufweist als am proximalen Ende. Beispielsweise ist es möglich, dass die Querschnittsfläche der einen Faser und/oder auch der mehreren Fasern und/oder des Lichtleiters am proximalen Ende im Wesentlichen, also im Rahmen der Messgenauigkeit, kreisrund ausgebildet sein kann, am distalen Ende aber beispielsweise oval oder nierenförmig oder mit im Wesentlichen D-förmigen Querschnitt vorliegt. Es ist auch möglich, dass unterschiedliche lichtleitende Fasern unterschiedliche Querschnittsflächen aufweisen, wobei insbesondere am proximalen Ende die Querschnittsfläche rund sein kann, im distalen Ende aber für eine oder mehrere Fasern oval, für andere nierenförmig. Es sind auch andere Querschnittsflächen denkbar, beispielsweise rechteckige oder annähernd rechteckige Querschnittsflächen, insbesondere am distalen Ende, oder allgemein polygonale Querschnittsflächen. Es ist weiter möglich, dass die Querschnittsfläche der einen Faser und/oder auch der mehreren Fasern und/oder des Lichtleiters am proximalen und/oder am distalen Ende eine Form aufweist, welche von mindestens zwei Linien begrenzt ist, die voneinander unterschiedliche Krümmungsradien aufweisen und/oder die als Differenzfläche von zwei einander nur teilweise überlappenden Kreisen und/oder Ellipsen ausgebildet ist. Insbesondere kann die Querschnittsfläche als Kreissegment ausgestaltet sein, wobei im Falle des Kreissegments ein Krümmungsradius unendlich ist, also eine im Rahmen der Messgenauigkeit gerade Linie ist. Eine solche Querschnittsfläche, welche als Kreissegment ausgebildet ist, kann wiederum auch als eine D-förmige Querschnittsfläche bezeichnet werden oder als eine im Wesentlichen D-förmige Querschnittsfläche.

Insbesondere ein nahezu D-förmiger Querschnitt bietet hier eine hohe Ausnutzung der zur Verfügung stehenden Kavitäten und kann somit zu einem erhöhten Lichtstrom bzw. zu einer erhöhten Beleuchtungsstärke am distalen Ende des Endoskops führen. Unter einem im Wesentlichen D-förmigen Querschnitt oder einer im Wesentlichen D-förmigen Querschnittsfläche wird im Rahmen der vorliegenden Offenbarung insbesondere eine Fläche verstanden, welche als Kreissegment ausgebildet ist.

Eine solche Ausgestaltung kann insbesondere dann vorteilhaft sein, um eine räumliche besonders günstige Anordnung der Faser und/oder der Fasern und/oder des Lichtleiters in Bezug auf den Kamerachip zu gewährleisten.

Allgemein ist es möglich, dass die zumindest eine lichtleitende Faser und/oder dass die lichtleitenden Fasern zumindest abschnittsweise eine Querschnittsfläche aufweisen, welche von einer zumindest im Rahmen der Messgenauigkeit von der runden Form abweichende Form aufweist. Dies kann vorteilhaft sein, um besonders effiziente, beispielsweise raumsparende, Anordnungen einzelner Elemente im zweiten Bauteil des Endoskops zu ermöglichen.

Gerade im distalen Ende des Lichtleiters kann dies vorteilhaft sein.

Gemäß einer Ausführungsform weist daher die zumindest eine lichtleitende Faser und/oder weisen die mehreren lichtleitenden Fasern zumindest am distalen Ende des Lichtleiters einen Querschnitt mit einer abgeflachten Form mit einem Aspektverhältnis von mindestens 1,5 : 1 und/oder einem ovalen Querschnitt und/oder einem nierenförmigen Querschnitt auf, und/oder eine Querschnittsfläche, welche von mindestens zwei Linien begrenzt ist, die voneinander unterschiedliche Krümmungsradien aufweisen, und/oder die als Differenzfläche von zwei einander nur teilweise überlappenden Kreisen und/oder Ellipsen ausgebildet ist.

Gemäß einer weiteren Ausführungsform ist die numerische Apertur der einen oder der mehreren lichtleitenden Fasern mindestens 0,7, bevorzugt mindestens 0,8 und besonders bevorzugt mindestens 0,85. Vorzugsweise umfasst der Kern der einen oder der mehreren lichtleitenden Fasern ein glasiges Material, dessen Zusammensetzung ausgewählt ist aus den vorstehend aufgeführten Glaszusammensetzungen und Glaszusammensetzungsbereichen für Kerngläser. Insbesondere kann der Kern der Glasfaser überwiegend, also zu mindestens 50 Gew.-%, oder im Wesentlichen, also zu mindestens 90 Gew.-%, oder sogar vollständig aus einem solchen glasigen Material bestehen.

Eine Ausführungsform, bei welcher der Kern der einen oder der mehreren lichtleitenden Fasern ein solches glasiges Material umfasst, ist vorteilhaft, weil auf diese Weise eine sehr gute Ausleuchtung des Kamera-Gesichtsfeldes (hier insbesondere für sogenannte C-MOS-Kameras mit z.B. 1 x 1 mm² Fläche) realisiert werden kann.

Gemäß einer weiteren Ausführungsform ist die eine lichtleitende Faser oder sind die mehreren lichtleitenden Fasern so ausgebildet, dass das Kern- und/oder das Mantelglas der einen lichtleitenden Faser oder der mehreren lichtleitenden Fasern bis auf unvermeidliche Spuren frei ist von Blei und/oder sonstigen Schwermetallen sowie frei von Antimon und/oder Arsen und/oder von sonstigen kritischen Elementen wie Cr(VI) ist.

Ein weiterer Aspekt der vorliegenden Erfindung betrifft ein Einweg-Endoskopsystem mit einem ersten Bauteil und steril einzeln verpackten zweiten Bauteilen, welche vorzugsweise als Schäfte ausgebildet sind oder sein können, welche nach der Entnahme aus ihrer sterilen Verpackung lösbar mit dem ersten Bauteil koppelbar sind, um ein Endoskop, insbesondere ein Endoskop nach Ausführungsformen der vorliegenden Offenbarung, zu erhalten.

Unter einem Schaft wird im Rahmen der vorliegenden Offenbarung ein zweites Bauteil eines Endoskops verstanden, welches einen im Vergleich zu seiner Länge nur geringe Querschnittsfläche aufweist. Mit anderen Worten ist der Schaft also im Vergleich zur Länge dünn ausgebildet. Eine solche Ausgestaltung eines zweiten Bauteils als Schaft ist vorteilhaft, gerade wenn nur sehr schwer zugängliche Bereiche mittels des Endoskops untersucht werden und/oder für Anwendungen in der Medizintechnik.

Ein Vorteil des Endoskop-Systems nach der vorliegenden Offenbarung ist es, für kurz aufeinander abfolgende Untersuchungen bereits steril verpackte zweite Bauteile, insbesondere Schäfte, vorliegen zu haben, sodass rasche mehrere Bereiche untersucht werden können oder, bei medizinischen Untersuchungen, mehrere Untersuchungen von unterschiedlichen Patienten kurz hintereinander unter Sicherstellung einer ausreichenden Hygiene durchgeführt werden können. Daher ist es gerade für das Endoskop-System nach der vorliegenden Offenbarung vorteilhaft, dass die zweiten Bauteile lösbar mit dem ersten Bauteil koppelbar sind, um auf diese Weise die Vorteile eines Einweg-Endoskops zu ermöglichen, wobei gleichzeitig die Teile des Endoskopsystems, welche beispielsweise bei medizinischen Untersuchungen oder sonstigen medizinischen Einsatzzwecken nicht zwingend sterilisiert vorliegenden müssen, in einem mehrfach nutzbaren, ersten Bauteil untergebracht sind. Auf diese Weise ist es nämlich möglich, beispielsweise eine Beleuchtung mit Laserlicht auch für Einweg-Endoskope zu ermöglichen.

Gemäß einer Ausführungsform ist das zweite Bauteil als zumindest abschnittsweise flexibler Schaft vorgesehen, welcher eine flexible Ummantelung mit einem Schlauch oder Geflechtschlauch oder Schrumpfschlauch umfasst, welche den Lichtleiter mit seiner mindestens einen lichtleitenden Faser sowie eine Versorgungsleitung zur elektrischen Speisung des Kamerachips und vorzugsweise mindestens eine Rücksignalleitung zu einer Daten- und/oder Bildweiterverarbeitungseinheit, welche insbesondere als Bestandteil im ersten Bauteil vorliegen kann, zumindest abschnittsweise umschließt. Eine solche Ausgestaltung, insbesondere mit einem flexiblen Schaft, ist insbesondere für medizinische Anwendungen geeignet.

Gemäß einer weiteren Ausführungsform ist das zweite Bauteil ausgebildet als zumindest abschnittsweise starrer Schaft vorgesehen, welcher eine starre Ummantelung mit einer Hülse umfasst, welche den Lichtleiter mit seiner mindestens einen lichtleitenden Faser sowie eine Versorgungsleitung zur elektrischen Speisung des Kamerachips und vorzugsweise eine Rücksignalleitung, vorzugsweise zu einer Daten- und/oder Bildweiterverarbeitungseinheit, welche insbesondere als Bestandteil im ersten Bauteil vorliegen kann, umschließt. Eine solche Ausgestaltung kann insbesondere vorteilhaft sein, weil auf diese Weise die vom zweiten Bauteil, das hier als starr ausgebildeter Schaft vorgesehen ist, umfassten Elemente besser gegenüber mechanischen Belastungen geschützt sein können.

Die vorliegende Offenbarung betrifft allgemein eine Lichtquelle für ein Endoskop, insbesondere eine Lichtquelle für ein Endoskop nach Ausführungsformen der vorliegenden Offenbarung. Die Lichtquelle für ein Endoskop, insbesondere für ein Endoskop nach Ausführungsformen der vorliegenden Offenbarung, umfasst einen Laser zur Abgabe von Primärlicht, vorzugsweise zur Abgabe von blauem und/oder ultravioletten Licht, sowie mindestens einen dem Laser zugeordneten Konverter und einen Lichtleiter mit einer oder mehreren lichtleitenden Fasern, wobei der Laser so angeordnet ist, dass das Licht des Lasers eine Fläche des Konverters zumindest teilweise bestrahlt und das proximale Ende des Lichtleiters mit seiner mindestens einen lichtleitenden Faser das konvertierte und gestreute bzw. das ausgesendete Licht des Konverters aufnimmt.

Eine solche Ausgestaltung einer Lichtquelle ermöglicht eine verbesserte Koppeleffizienz, da es nämlich möglich ist, beispielsweise eine lichtleitende Faser oder auch mehrere lichtleitende Fasern mit einer hohen numerischen Apertur gegenüber Luft zu verwenden. Auch kann auf diese Weise der Anregungslaser räumlich von sonstigen Elementen des Endoskops entkoppelt werden, sodass beispielsweise auch eine große Aufheizung dieser Elemente durch den Laser verhindert werden kann.

Allgemein, ohne Beschränkung auf das vorstehend beschriebene Beispiel, ist es weiterhin möglich, dass die Lichtquelle weitere Bestandteile bzw. Bauteile umfassen kann. Insbesondere kann die Lichtquelle optische Elemente umfassen, welche beispielsweise Laserlicht lenken und/oder modifizieren können, insbesondere welche das Laserlicht kollimieren. Beispielsweise können solche Bauteile als diffraktive optische Elemente vorliegen (DOE) oder ausgestaltet sein. Eine solche Ausgestaltung kann beispielsweise dann vorteilhaft sein, wenn das DOE so ausgestaltet ist, dass zumindest eine Fläche des Konverters vollständig beleuchtet wird oder so, dass das Licht des Lasers auf mehrere unterschiedliche Konverter bzw. Konverterelemente gelenkt wird. Es ist aber auch möglich, dass optische Elemente vorgesehen sind, welche nicht das Primärlicht kollimieren und/oder lenken und/oder modifizieren, sondern optische Elemente, welche das Sekundärlicht, also das konvertierte und/oder gestreute Licht lenken und/oder modifizieren und/oder kollimieren.

Insbesondere kann ein distal angeordneter Diffusor vorgesehen sein, der das von der Faser geleitete Licht in einen breiteren Raumwinkel abgibt und damit ein größerer Gewebebereich ausgeleuchtet werden kann.

Gemäß einer weiteren Ausführungsform umfasst die Lichtquelle eine lichtleitende Faser zur Zuführung des Laserlichts zum Konverter, wobei das Auskoppelende der lichtleitenden Faser und das Einkopplungsende des Lichtleiters auf die gleiche Fläche des Konverters gerichtet sind, sodass der Konverter in Remission betrieben wird und die Leitungsrichtung des Lichts in der lichtleitenden Faser zur Leitung des Laserlichts entgegengesetzt zur Leitungsrichtung des vom Konverter in den Lichtleiter eingekoppelten Lichts ist. Auf diese Weise kann verhindert werden, dass direktes Laserlicht in den Lichtleiter des Endoskops eingekoppelt wird, was unter Sicherheitsgesichtspunkten vorteilhaft ist. Insbesondere bei einer Fehlfunktion des Konverters kann mit einer solchen Anordnung verhindert werden, dass das recht intensive Laser-Licht oder auch die Primärstrahlung der Laser-Lichtquelle direkt zum Gewebe des Patienten gelangt. Üblicherweise, so sei angemerkt, sind die Konverter in dieser Anordnung wärmeleittechnisch mit einer Wärmesenke, z.B. in Form eines Kühlkörpers als passives Bauteil oder mit einer aktiven Kühlung, verbunden. Zudem ist diese zusätzlich als sogenannte Strahlfalle ausgebildet, falls der Konverter beschädigt oder gar komplett zerstört werden sollte.

Gemäß einer weiteren Ausführungsform umfasst der Konverter zwei Konverterelemente, wobei die Konverterelemente jeweils ein keramisches Konvertermaterial enthalten, wobei vorzugsweise die Konverterelemente unterschiedliche Konvertermaterialien enthalten, sodass die Konverterelemente das Laserlicht in Licht unterschiedlicher spektraler Zusammensetzung konvertieren, wobei zumindest ein Laser vorgesehen ist, welcher beide Konverterelemente mit jeweils einem Laserstrahl bestrahlt.

Denkbar sind auch einzelne Konverter, die aus einer Mischung zweier Konvertermaterialien bestehen, welche Licht mit unterschiedlicher Wellenlänge emittieren.

Solche Ausgestaltungen ermöglichen es, einen besonders guten CRI zu erhalten, also Farben besonders gut wiedergeben zu können.

Vorzugsweise weist der Lichtleiter zwei Einkoppelenden auf, wobei die Konverterelemente so angeordnet sind, dass das von den Konverterelementen abgegebene Licht in jeweils eines der Einkoppelenden eingekoppelt wird.

Allgemein kann die Lichtquelle so ausgestaltet sein, dass sie eine Vielzahl von Konverterelementen umfasst, wobei der Lichtleiter so ausgestaltet ist, dass er eine Vielzahl von Einkoppelende aufweist, wobei die Konverterelemente so angeordnet sind, dass das von den Konverterelementen abgegebene Licht jeweils in eines der Einkoppelenden eingekoppelt wird, wobei die Anzahl der Konverterelemente der Anzahl der Einkoppelenden entspricht, sodass vorzugsweise jedem Konverterelement ein eigenes Einkoppelende zugeordnet ist.

Mit solchen Ausgestaltungen sind besonders hohe CRI-Werte erzielbar. Denn mit den lichtleitenden Fasern ist die erfolgte Farbmischung tatsächlich additiv.

Besonders vorteilhaft kann es sein, wenn das Verhältnis der in den Lichtleiter eingekoppelten Lichtströme einstellbar ist. Auf diese Weise kann der Farbort des erzeugten Lichts der Lichtquelle auf besonders einfache Weise angepasst werden. -Daher weist gemäß einer Ausführungsform die Lichtquelle eine Einrichtung zur Einstellung des Verhältnisses der in den Lichtleiter eingekoppelten Lichtströme von beiden Konverterelementen bzw. von allen von der Lichtquelle umfassten Konverterelementen auf.

Nach einem ersten Aspekt ist daher ein Endoskop mit einem ersten Bauteil und einem zweiten Bauteil offenbart, wobei im ersten Bauteil eine Lichtquelle eingebaut ist, und wobei das zweite Bauteil ein mit dem ersten Bauteil verbundenes proximales Ende, vorzugsweise ein lösbar verbundenes proximales Ende, und ein distales Ende aufweist, wobei im distalen Ende ein Element zur Bildaufnahme, wie ein Kamerachip oder ein faseroptisches Element, angeordnet ist, und wobei im zweiten Bauteil ein Lichtleiter mit zumindest einer lichtleitenden Faser verläuft, um Licht der Lichtquelle vom proximalen Ende zum distalen Ende zu leiten und am distalen Ende abzugeben, sowie vorzugsweise eine Versorgungsleitung zur elektrischen Speisung des Kamerachips, und wobei die Lichtquelle mindestens einen Laser zur Abgabe von Primärlicht umfasst, sowie einen Konverter, welcher das Licht des Lasers zumindest teilweise in Licht einer anderen Wellenlänge umwandelt und abgibt, wobei der Konverter so mit dem -am ersten Bauteil verbundenen proximalen Ende des zweiten Bauteils gekoppelt ist, dass das vom Konverter umgewandelte und abgegebene Licht in den Lichtleiter eingekoppelt wird.

Nach einem zweiten Aspekt ist das Endoskop nach dem ersten Aspekt offenbart, wobei der Konverter ein keramisches Konvertermaterial umfasst.

Nach dem dritten Aspekt ist das Endoskop nach dem zweiten Aspekt offenbart, wobei der Konverter mindestens zwei keramische Konvertermaterialien aufweist, welche das Laserlicht in Licht unterschiedlicher spektraler Zusammensetzungen konvertieren.

Nach dem vierten Aspekt ist das Endoskop nach dem dritten Aspekt offenbart, wobei der Konverter zwei Konverterelemente umfasst, wobei die Konverterelemente jeweils eines der keramischen Konvertermaterialien enthalten, so dass die Konverterelemente das Laserlicht in Licht unterschiedlicher spektraler Zusammensetzung konvertieren.

Nach dem fünften Aspekt ist das Endoskop nach einem der Aspekt 1 bis 4 offenbart, aufweisend wenigstens eines der folgenden Merkmale:
- der Konverter ist so optisch mit dem Lichtleitergekoppelt, dass vom Konverter remittiertes, also konvertiertes und/oder gestreutes und/oder reflektiertes, Licht in den Lichtleiter eingekoppelt ist und/oder wird und/oder zumindest einkoppelbar ist,
- der Laser ist so angeordnet und auf den Konverter gerichtet, dass ausschließlich vom Konverter konvertiertes und/oder gestreutes und/oder reflektiertes Licht in den Lichtleiter eingekoppelt ist und/oder wird und/oder zumindest einkoppelbar ist,
- der Laser ist so angeordnet, dass das Licht des Lasers im Wesentlichen entgegen der Lichtabstrahlrichtung des vom Konverter konvertierten und in den Lichteiter eingekoppelten Lichts auf den Konverter gerichtet wird und/oder gerichtet ist und/oder auf diesen lenkbar ist.

Nach dem sechsten Aspekt ist das Endoskop nach einem der fünf vorhergehenden Aspekte offenbart, wobei der Lichtleiter maximal zwanzig, vorzugsweise maximal zehn lichtleitende Fasern aufweist.

Nach dem siebten Aspekt ist das Endoskop nach einem der Aspekt 1 bis 6 offenbart, wobei die eine lichtleitende Faser oder die mehreren lichtleitenden Fasern wenigstens eines der folgenden Merkmale aufweisen:
- einen Durchmesser im Bereich von 100 µm bis 1000 µm, vorzugsweise im Bereich 100 µm bis 600 µm und besonders bevorzugt im Bereich von 150 µm bis 400 µm, wobei die lichtleitenden Einzelfasern auch unterschiedliche Durchmesser aufweisen können,
- die eine lichtleitende Faser oder die mehreren lichtleitenden Fasern sind Stufenindex-Glasfasern, wobei vorzugsweise die eine lichtleitende Faser und oder die mehreren lichtleitenden Fasern Stufenindex-Glasfasern umfassend eine Glaszusammensetzung, die bis auf unvermeidliche Spuren frei ist von Blei und/oder sonstigen Schwermetallen sowie frei von Antimon und/oder Arsen und/oder von sonstigen kritischen Elementen wie Cr(VI) ist, sind,
- die numerische Apertur (NA) gegen Luft der einen oder der mehreren lichtleitenden Fasern ist mindestens 0,7, bevorzugt mindestens 0,8, besonders bevorzugt mindesten 0,85,

Nach dem achten Aspekt ist das Endoskop nach dem siebten Aspekt offenbart, wobei die eine oder die mehreren lichtleitenden Fasern eine an deren Mantelfläche polymerbasierte Beschichtung oder eine Schutzhülle aus einem polymerbasiertem Schlauchmaterial aufweisen,
wobei bevorzugt
die Beschichtung aus einer Acrylat-, Polyamid-, Polyurethan-, Polyimid-, Epoxi-, Ethylen-Tetrafluorethylen-Copolymer- oder Poly-Xylol-basierten Verbindung oder aus einer Mischung dieser Verbindungen besteht
   und/oder
wobei die Beschichtung mittels Tauchen, Besprühen, Extrudieren oder Abscheidung bei niedrigem Druck auf die eine oder die mehreren lichtleitenden Fasern unmittelbar nach dem Ziehen der Fasern aufbringbar ist oder aufgebracht ist
wobei vorzugsweise die Beschichtung eine Schichtdicke von 10 µm bis 100 µm, vorzugsweise von 20 µm bis 50 µm aufweist.

Nach dem neunten Aspekt ist das Endoskop nach dem achten Aspekt offenbart, wobei die Beschichtung mindestens eine weitere äußere Beschichtung aufweist, welche aus PMMA, Polyamid, Polyimid oder aus einem fluorierten Polymer, wie einem Ethylen-Tetrafluorethylen-Copolymer, oder aus einer Mischung daraus bestehen kann.

Nach dem zehnten Aspekt ist das Endoskop nach einem der Aspekte 1 bis 9 offenbart, wobei die eine lichtleitende Faser oder die mehreren lichtleitenden Fasern am proximalen Ende in einer Einkoppelhülse angeordnet sind.

Nach dem elften Aspekt ist das Endoskop nach einem der Aspekte 1 bis 10 offenbart, wobei die lichtleitenden Fasern am proximalen Ende heiß-verschmolzen angeordnet sind.

Nach dem zwölften Aspekt ist das Endoskop nach einem der Aspekte 1 bis 11 offenbart, wobei die zumindest eine lichtleitende Faser und/oder die mehreren lichtleitenden Fasern und/oder der Lichtleiter am distalen Ende gegenüber dem proximalen Ende verformt ist.

Nach dem dreizehnten Aspekt ist das Endoskop nach einem der Aspekte 1 bis 12 offenbart, wobei zumindest eine lichtleitende Faser und/oder die mehreren lichtleitenden Fasern zumindest am distalen Ende des Lichtleiters einen Querschnitt mit einer abgeflachten Form mit einem Aspektverhältnis von mindestens 1,5: 1 aufweist, und/oder wobei die Form der Querschnittsfläche an die zwischen Kamerachip und Außenkontur des distalen Endes verbleibende Fläche angepasst ist, insbesondere einen ovalen Querschnitt und/oder einen nierenförmigen Querschnitt und/oder einen Querschnitt in Form eines Kreissegments aufweist.

Nach dem vierzehnten Aspekt ist ein Einweg-Endoskopsystem mit einem ersten Bauteil und steril einzeln verpackten zweiten Bauteilen, welche vorzugsweise als Schäfte ausgebildet sind oder sein können, welche nach Entnahme aus ihrer sterilen Verpackung lösbar mit dem ersten Bauteil koppelbar sind, offenbart, um ein Endoskop nach einem der Aspekte 1 bis 13 zu erhalten.

Nach dem fünfzehnten Aspekt ist das Einweg-Endoskopsystem nach Aspekt 14 offenbart, wobei ein zweites Bauteil ausgebildet als zumindest abschnittsweise flexibler Schaft vorgesehen ist, welcher eine flexible Ummantelung mit einem Schlauch oder Geflechtschlauch oder Schrumpfschlauch umfasst, welche den Lichtleiter mit seiner mindestens einen lichtleitenden Faser sowie eine Versorgungsleitung zur elektrischen Speisung des Kamerachips und vorzugsweise mindestens eine Rücksignalleitung, vorzugsweise zu einer Daten und/oder Bildweiterverarbeitungseinheit, welche insbesondere als Bestandteil im ersten Bauteil vorliegen kann, zumindest abschnittsweise umschließt.

Nach dem sechzehnten Aspekt ist das Einweg-Endoskopsystem nach Aspekt 15 oder 14 offenbart, wobei ein zweites Bauteil ausgebildet als zumindest abschnittsweise starrer Schaft vorgesehen ist, welcher eine starre Ummantelung mit einer Hülse umfasst, welche den Lichtleiter mit seiner mindestens einen lichtleitenden Faser sowie eine Versorgungsleitung zur elektrischen Speisung des Kamerachips und vorzugsweise mindestens eine Rücksignalleitung zu einer Daten- und/oder Bildweiterverarbeitungseinheit, welche vorzugsweise als Bestandteil im ersten Bauteil vorliegen kann, umschließt.

Nach dem siebzehnten Aspekt ist eine Lichtquelle für ein Endoskop offenbart, insbesondere für ein Endoskop nach einem der Aspekte 1 bis 13, umfassend einen Laser zur Abgabe von Primärlicht, vorzugsweise zu Abgabe von blauem und/oder ultravioletten Licht, sowie mindestens einen dem Laser zugeordneten Konverter und einen Lichtleiter mit einer oder mehreren lichtleitenden Fasern, wobei der Laser so angeordnet ist, dass das Licht des Lasers eine Fläche des Konverters zumindest teilweise bestrahlt und das proximale Ende des Lichtleiters mit seiner mindestens einen lichtleitenden Faser das konvertierte und/oder gestreute und/oder das reflektierte Licht des Konverters aufnimmt.

Nach dem achtzehnten Aspekt ist die Lichtquelle nach Aspekt 17 offenbart, umfassend eine lichtleitende Faser zur Zuführung des Laserlichts zum Konverter, wobei das Auskoppelende der lichtleitenden Faser und das Einkopplungsende des Lichtleiters auf die gleiche Fläche des Konverters gerichtet sind, so dass der Konverter in Remission betrieben wird und die Leitungsrichtung des Lichts in der lichtleitenden Faser zur Leitung des Laserlichts entgegengesetzt zur Leitungsrichtung des vom Konverter in den Lichtleiter eingekoppelten Lichts ist.

Nach dem neunzehnten Aspekt ist die Lichtquelle nach einem der Aspekte 17 oder 18 offenbart, wobei der Konverter zwei Konverterelemente umfasst, wobei die Konverterelemente jeweils ein keramisches Konvertermaterial enthalten, wobei vorzugsweise die Konverterelemente unterschiedliche Konvertermaterialien enthalten, so dass die Konverterelemente das Laserlicht in Licht unterschiedlicher spektraler Zusammensetzung konvertieren, wobei zumindest ein Laser vorgesehen ist, welcher beide Konverterelemente jeweils mit einem Laserstrahl bestrahlt.

Nach dem zwanzigsten Aspekt ist die Lichtquelle nach Aspekt 19 offenbart, wobei der Lichtleiter zwei Einkoppelenden aufweist, wobei die Konverterelemente so angeordnet sind, dass das von den Konverterelementen abgegebene Licht in jeweils eines der Einkoppelenden eingekoppelt wird.

Nach dem einundzwangzigsten Aspekt ist die Lichtquelle nach einem der Aspekte 19 oder 20 offenbart, umfassend eine Einrichtung zur Einstellung des Verhältnisses der in den Lichtleiter eingekoppelten Lichtströme von beiden Konverterelemente.

Diese Anmeldung ist eine Teilanmeldung der europäischen Anmeldung EP 20209014.8 (EP 3831273), die hiermit in ihrer Gesamtheit in den Anmeldungstext aufgenommen ist. Der Anmelder behält sich das Recht vor, jeden in der ursprünglichen Anmeldung EP 20209014.8 (EP 3831273) offenbarten Gegenstand in der vorliegenden Teilanmeldung oder in einer oder mehrerer gegebenenfalls folgender weiterer Teilanmeldungen zu beanspruchen, unabhängig vom Gegenstand der beigefügten Ansprüche.

### Beschreibung der Zeichnungen

Im Folgenden wird die Erfindung anhand von Figuren weiter erläutert. Dabei bezeichnen gleiche Bezugszeichen die gleiche oder einander entsprechende Elemente. Es zeigen:
- Fig. 1: eine schematische und nicht maßstabsgetreue Abbildung eines Endoskops nach einer Ausführungsform,
- Fig. 2: schematische und nicht maßstabsgetreue Abbildungen von distalen Enden eines Endoskops, sowie
- Fig. 3 und 4: schematische und nicht maßstabsgetreue Abbildungen von Teilen von Lichtquellen gemäß Ausführungsformen.

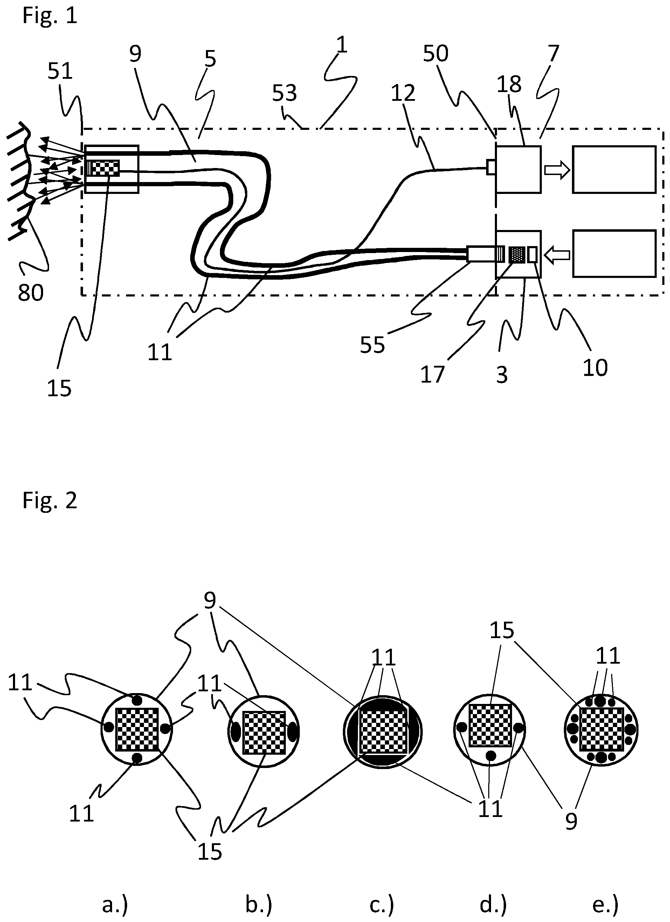

Fig. 1 ist eine schematische und nicht maßstabsgetreue Abbildung eines Endoskops 1 nach einer Ausführungsform. Das Endoskops 1 umfasst eine erstes Bauteil 7 und ein zweites Bauteil 5, wobei hier in der Darstellung das erste Bauteil rechts angeordnet ist und das zweite Bauteil 5 links. Das zweite Bauteil 5 umfasst ein mit dem ersten Bauteil 7 verbundenes proximales Ende 50. Es kann vorgesehen sein, dass das mit dem ersten Bauteil 7 verbundene proximale Ende 50 des zweiten Bauteils 5 so ausgestaltet ist, dass es lösbar ist. Insbesondere können also die beiden Bauteil 5 und 7 so ausgestaltet sein, dass sie mittels einer lösbaren Verbindung ausgestaltet vorliegen. Dies kann insbesondere dann von Vorteil sein, wenn ein Bauteil lediglich für einen Einmalgebrauch vorgesehen ist, das andere Bauteil, hier beispielsweise das erste Bauteil 7, Komponenten umfasst, welche für den Mehrfachgebrauch bestimmt sind, insbesondere solche, welche hochwertig und/oder hochpreisig sind. Insbesondere kann dies der Fall sein, wenn eine besondere Lichtquelle, wie beispielsweise eine Lichtquelle umfassend mindestens einen Laser, von einem der Bauteile, hier beispielsweise dem ersten Bauteil 7, umfasst ist.

Das zweite Bauteil weist weiterhin ein distales Ende 51 auf, wobei im distalen Ende 51 ein Kamerachip 15 zur Bildaufnahme angeordnet ist. Im zweiten Bauteil 5 verläuft weiterhin ein Lichtleiter 9 umfassend mindestens eine lichtleitende Faser 11. Diese ist ausgestaltet, um Licht einer Lichtquelle 3 vom proximalen Ende 50 zum distalen Ende 51 des Lichtleiters 9 zu leiten und am distalen Ende 51 abzugeben. Weiterhin verläuft im zweiten Bauteil 5 eine Versorgungsleitung (nicht dargestellt) zur elektrischen Speisung des Kamerachips 15.

Die Lichtquelle 3 umfasst mindestens einen Laser 10, der zu Abgabe von Primärlicht ausgelegt ist, sowie einen Konverter 17, welches das Licht des Lasers 10 zumindest teilweise in Licht einer anderen Wellenlänge umwandelt und abgibt. Der Konverter 17 ist so mit dem am ersten Bauteil 7 verbundenen proximalen Ende 50 des zweiten Bauteils 5 gekoppelt, dass das vom Konverter 17 umgewandelte und abgegebene Licht in den Lichtleiter 9 eingekoppelt wird.

Der Konverter 17 umfasst bevorzugt ein keramisches Konvertermaterial.

Der Konverter 17 kann dabei so ausgestaltet sein, dass er mindestens zwei keramische Konvertermaterialien aufweist, welche das Licht des Lasers 10 (oder Laserlicht) in Licht unterschiedlicher spektraler Zusammensetzungen konvertieren.

Allgemein, ohne Beschränkung auf das beispielhaft in Fig. 1 dargestellte Endoskop 1 bzw. die von dem beispielhaften Endoskop 1 nach Fig. 1 umfasste Lichtquelle 3 mit dem Laser 10, kann der Konverter 17 verstanden werden als umfassend ein Konverterelement (hier nicht dargestellt), welches das Konvertermaterial umfasst. Insbesondere kann dieses Konverterelement so ausgestaltet sein, dass es das Konvertermaterial, welches beispielsweise und hier insbesondere bevorzugt ein keramisches Material sein oder dieses umfassen kann, umfasst, welches beispielsweise in Form einer dünnen Materialschicht auf eine Unterlage aufgebracht ist, welche als Ableiter für die bei der Konversion des Primärlichts entstehende thermische Energie wirken kann. Eine solche Ausgestaltung ist insbesondere dann bevorzugt, wenn die Lichtquelle bzw. der Konverter in Remission betrieben wird.

Gemäß einer Ausführungsform des Endoskops ist der Konverter 17 so optisch mit dem Lichtleiter 9 gekoppelt, dass vom Konverter 17 remittiertes Licht in den Lichtleiter 9 eingekoppelt ist und/oder wird und/oder zumindest einkoppelbar ist. Vorzugsweise kann der Laser 10 so angeordnet und auf den Konverter 17 gerichtet sein, dass ausschließlich vom Konverter 17 konvertiertes und/oder gestreutes Licht in den Lichtleiter 9 eingekoppelt ist und/oder wird und/oder zumindest einkoppelbar ist. Eine solche Ausgestaltung ist insbesondere unter Sicherheitsgesichtspunkten sinnvoll, wenn verhindert werden soll, dass hochenergetisches Laserlicht beispielsweise auf eine Gewebeoberfläche 80, welche hier in Fig. 1 beispielhaft links dargestellt ist, gelangt.

Vorteilhaft kann es dabei vorgesehen sein, dass der Laser 10 so angeordnet ist, dass das Licht des Lasers 10 entgegen der Lichtabstrahlrichtung des vom Konverter 17 konvertierten und in den Lichtleiter 9 eingekoppelten Lichts auf den Konverter 17 gerichtet wird und/oder gerichtet ist und/oder auf diesen lenkbar ist.

Insbesondere unter Montagegesichtspunkten, gerade wenn das zweite Bauteil 5 nur für den Einzelgebrauch vorgesehen ist, kann es vorteilhaft sein, wenn der Lichtleiter 9 maximal zehn lichtleitende Faser 11 aufweist. Es ist aber allgemein möglich, dass bis zu einigen hundert Einzelfasern 11 in einem Lichtleiter 9 sind, wobei dies von den entsprechenden Faserdurchmessern und der resultierenden bzw. adressierten Dicke des Faserbündels und mithin des Lichtleiters 9 abhängt und entsprechend die Zahl der Fasern 11 ausgewählt werden kann.

Typische Faserdurchmesser (oder Faserdicken) von lichtleitenden Fasern 11 können in bevorzugter Weise in einem Bereich von 100 µm bis 1000 µm, bevorzugt bis 600 µm, liegen, wobei der maximale Faserdurchmesser besonders vorzugsweise in einem Bereich von 150 µm bis 400 µm liegt. Es sind aber auch dünnere Fasern mit Durchmessern von 30 µm, 50 µm oder 70 µm denkbar.

Gemäß einer Ausführungsform ist die eine lichtleitende Faser 11 oder sind die mehreren lichtleitenden Fasern 11 als Stufenindex-Glasfasern ausgebildet.

Vorzugsweise kann die eine lichtleitende Faser 11 und/oder können die mehreren lichtleitenden Fasern 11 so ausgebildet sein, dass die numerische Apertur (NA) gegen Luft der mindestens einen Faser 11 und/oder der mehreren lichtleitenden Fasern 11 mindestens 0,7, bevorzugt mindestens 0,8 und besonders bevorzugt mindestens 0,85 ist. Dies ist besonders günstig, um einen hohen CRI (Color rendering index) zu erhalten.

Insbesondere unter Montagegesichtspunkten kann es günstig sein, wenn die mindestens eine lichtleitende Faser 11 oder die mehreren lichtleitenden Fasern 11 am proximalen Ende 50 des Lichtleiters 9, wie in Fig. 1 schematisch auch dargestellt, in einer Einkoppelhülse 55 angeordnet sind.

Das zweite Bauteil 5 kann beispielsweise als zumindest abschnittsweise flexibler Schaft vorgesehen sein oder auch als zumindest abschnittsweise starrer Schaft. Das zweite Bauteil kann beispielsweise eine Ummantelung 53 umfassen, wie beispielhaft in Fig. 1 dargestellt. Für den Fall, dass das Bauteil 5 als zumindest abschnittsweise flexibler Schaft ausgebildet ist, ist die Ummantelung 53 flexible ausgestaltet, insbesondere mit einem Schlauch oder Geflechtschlauch oder mit einem Schrumpfschlauch. Für den Fall der Ausbildung des zweiten Bauteils 5 als zumindest abschnittsweise starrer Schaft ist die Ummantelung 53 vorzugsweise starr ausgebildet und umfasst eine Hülse. Allgemein, ohne Beschränkung auf das hier beispielhaft dargestellte Beispiel, umschließt die Ummantelung 53 den Lichtleiter 9 mit der mindestens einen Faser 11, eine Versorgungsleitung zur elektrischen Speisung des Kamerachips 15 und vorzugsweise mindestens eine Rücksignalleitung 12, vorzugsweise eine Leitung zu einer daten- und/oder Bildweiterverarbeitungseinheit 18, welche insbesondere als Bestandteil im ersten Bauteil 7 vorliegen kann, zumindest abschnittsweise.

Als ein besonders bevorzugtes Ausführungsbeispiel erweist sich eine Anordnung mit sieben ca. 200 µm dicken lichtleitenden Fasern 11, welche als sogenannte Weitwinkelfaser mit einer NA > 0,85 ausgeführt sind, wobei die sieben lichtleitenden Fasern 11 um den Kamerachip 15 angeordnet sind, und am proximalen Ende in einer gemeinsamen Einkoppelhülse 55 verklebt sind. Alternativ können diese sieben lichtleitenden Fasern 11 in der Einkoppelhülse 55 auch heiß-verschmolzen sein. Allgemein ist es aber auch möglich und kann sogar bevorzugt sein, wenn am proximalen Ende heiß-verschmolzene Fasern hülsenlos vorliegen.

Fig. 2 zeigt schematisch und nicht maßstabsgetreu in Fig. 2a bis 2e Abbildungen von distalen Enden 51 eines zweiten Bauteils 5 eines Endoskops 1. Das distale Ende 51 umfasst jeweils den Lichtleiter 9 umfassend hier jeweils mehrere Fasern 11 sowie jeweils einen Kamerachip 15.

In Fig. 2a sind vier Fasern 11 angeordnet, welche einen im Rahmen der Messgenauigkeit runden Querschnitt aufweisen. Diese sind hier so um den Kamerachip 15, welcher hier beispielhaft als eine annähernd quadratische Form aufweist, angeordnet, dass jeweils an einer Seite des Kamerachips 15 eine Faser 11 ist. In Fig. 2d sind hingegen lediglich auf drei Seiten des Kamerachips 15 Fasern 11 angeordnet.

In der Fig. 2b sind lediglich zwei Fasern 11 auf zwei Seiten des Kamerachips 15 angeordnet. Hier ist der Querschnitt der lichtleitenden Fasern 11 nicht rund, sondern vielmehr oval bzw. elliptisch ausgebildet. Insbesondere können die lichtleitenden Fasern 11 dabei so ausgebildet sein, dass sie am distalen Ende 51, wie hier, gegenüber dem proximalen Ende 50 - hier nicht dargestellt - verformt sind. Insbesondere ist es möglich, dass die lichtleitenden Fasern 11 am proximalen Ende 50 einen runden Querschnitt aufweisen, am distalen Ende aber, wie hier, verformt vorliegen. Dies kann vorteilhaft sein, um die Fasern 11 um den Kamerachip herum anzuordnen.

Vorzugsweise können die lichtleitenden Fasern 11 und/oder die zumindest eine lichtleitende Faser 11 zumindest am distalen Ende 51, wie hier beispielhaft dargestellt, einen Querschnitt mit einer abgeflachten Form, insbesondere mit einem Aspektverhältnis von mindestens 1,5 : 1 und/oder einen ovalen Querschnitt und/oder einen nierenförmigen Querschnitt aufweisen. Andere Querschnittsformen, beispielsweise Polygone, sind denkbar, jedoch sind gerade abgeflachte Formen besonders vorteilhaft hinsichtlich der Anordnung der lichtleitenden Fasern 11 um den Kamerachips 15 herum. Fig. 2c zeigt eine Anordnung, bei denen vier Fasern, welche einen im Wesentlichen D-förmigen Querschnitt am distalen Ende aufweisen, um den Kamerachip 15 herum angeordnet sind.

Sowohl die in Fig. 2b als auch die in Fig. 2c dargestellten distalen Enden der Fasern 11 können beispielsweise durch einen Heiß-Umformprozess in der dargestellten Weise so verformt werden, dass die entsprechenden in den Figuren 2a bis 2d gezeigten Querschnittsflächen bzw. Querschnitte ausgebildet werden. Dabei wird die Faser 11 über deren Verarbeitungstemperatur in einer Form aufgeheizt und dann unter Druck verformt. Bedingt durch die Viskosität des Fasermaterials lassen sich natürlich nicht perfekte Geometrien nachbilden. So wird ein im Wesentlicher D-förmiger Querschnitt an den spitz zulaufenden Ecken kleinere Verrundungen aufweisen. Grundsätzlich kann eine derartige Formgebung sowohl bei Glasfasern, Quarz-Fasern oder auch Kunststofffasern angewendet werden, wobei die Umformtemperatur dem jeweiligen Material anzupassen ist. Bei Kunststofffasern (POFs) beträgt diese typischerweise 150° C bis 300° C, bei Glasfasern typischerweise zwischen 500° C und 800° C, je nach Glas-Type, und bei Quarz-Fasern bis zu 2000° C.

Fig.2e zeigt eine 12er-Anordnung, wie bereits zuvor beschrieben wurde. Hier sind insgesamt vier dickere Fasern 11 mit acht dünneren Fasern derart gruppiert, dass pro Kavität (Segment) die dicke Faser 11 in der Mitte der Kavität und die beiden dünneren Fasern 11 jeweils rechts und links von der dicken Faser 11 angeordnet ist. Damit kann trotz der vergleichsweise wenigen Fasern 11 bereits eine gute Flächennutzung der Kavität und damit ein vergleichsweise hoher Lichtstrom erzielt werden. Derartige Beispiele lassen sich z.B. auch auf eine 20er-Anordng mit 20 einzelnen Fasern 11, d.h. 5 Fasern 11 je Kavität ausweiten, bei denen es idealerweise 3 Durchmesserabstufungen bei den Fasern 11 gibt.

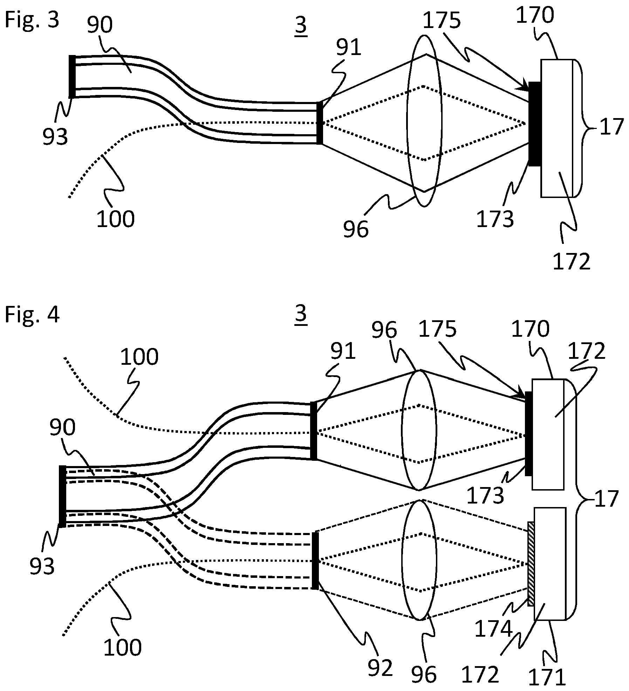

Schließlich zeigen Figuren 3 und 4 zwei schematische und nicht maßstabsgetreue Abbildungen eines Abschnitts oder Teils von einer Lichtquelle 3.

Die Lichtquelle 3 für ein Endoskop 1, insbesondere für ein Endoskop nach der vorliegenden Offenbarung, umfasst einen Laser 10 (nicht dargestellt) zur Abgabe von Primärlicht, vorzugsweise zur Abgabe von blauem und/oder ultravioletten Licht, sowie mindestens einen dem Laser zugeordneten Konverter 17 und einen Lichtleiter 90. Der Konverter 17 ist hier so ausgebildet, dass er ein erstes Konverterelement 170 umfassend ein keramisches Konvertermaterial 173 umfasst. Das Konverterelement 170 ist hierbei so ausgebildet, dass das keramische Konvertermaterial 173 als Materialschicht auf einer vom Konverterelement ebenfalls umfassten Unterlage oder auch Wärmesenke (oder heat sink) 172, welche beispielsweise zur Ableitung von thermischer Energie aufgrund der Konversion des Laserlichts ausgebildet sein kann, vorliegt. Weiterhin ist ein Lichtleiter 90 vorgesehen, welcher eine oder mehrere lichtleitende Fasern 11 umfasst. Der Laser 10 (nicht dargestellt) ist hierbei so angeordnet, dass das Licht des Lasers 10 eine Fläche des Konverters 17, nämlich insbesondere die Fläche 175, welche zumindest teilweise aus dem Konvertermaterial 173 gebildet wird, zumindest teilweise bestrahlt und das proximale Ende des Lichtleiters mit der mindestens einen lichtleitenden Faser 11 das konvertierte und/oder gestreute und/oder das ausgesendete Licht des Konverters 17 aufnimmt.

Gemäß der Abbildung in Fig. 3 kann dabei vorgesehen sein, dass eine lichtleitende Faser 100 das Laserlicht zum Konverter 17 zuführt. Das Auskoppelende der lichtleitenden Faser 100 und das Einkopplungsende 91 des Lichtleiters 90 sind in diesem Fall vorzugsweise auf die gleiche Fläche 175 des Konverters 17 gerichtet, wie beispielhaft in Fig. 3 dargestellt, sodass der Konverter in Remission betrieben wird. Die Leitungsrichtung des Lichts in der lichtleitenden Faser 100 ist in diesem Fall entgegengesetzt zur Leitungsrichtung des von Konverter in den Lichtleiter 90 eingekoppelten Lichts ist. Vorteilhaft kann vorgesehen sein, dass der Lichtleiter 90 am distalen Ende 93 eine Schnittstelle zum Lichtleiter 9 des zweiten Bauteils 5 eines Endoskops aufweist. Die Lichtquelle 3 kann weiterhin, wie hier im Form einer Linse 96 dargestellt, optische Elemente aufweisen, beispielsweise zur Strahlformung, zur Fokussierung und/oder Kollimation, insbesondere sogenannte diffraktive optische Elemente.

Fig. 4 zeigt eine weitere Abbildung eines Abschnitts oder Teils einer Lichtquelle 3 umfassend einen hier nicht dargestellten Laser 10. Der Konverter 17 umfasst hier zwei Konverterelemente 170, 171. Hier umfasst das Konverterelement 170 ein erstes Konvertermaterial 173, insbesondere ein keramisches Konvertermaterial 173, und das Konverterelement 171 umfasst ein zweites Konvertermaterial 174, insbesondere ein keramisches Konvertermaterial 174, wobei die Konvertermaterialien 173 und 174 hier so ausgebildet sind, dass sie unterschiedlich sind, sodass die Konverterelemente 170 und 171 das Laserlicht in Licht unterschiedlicher spektraler Zusammensetzung konvertieren. Beispielsweise kann das Konvertermaterial 173 als sogenannter "roter Phosphor" vorgesehen sein und das Konvertermaterial 174 als sogenannter "gelber Phosphor". Eine solche Ausgestaltung ist insbesondere dann vorteilhaft, um den sogenannten CRI zu optimieren, insbesondere einen CRI von mehr als 80 zu erzielen.

Allgemein kann die Lichtquelle 3 eine Vielzahl von Konverterelementen 170, 171 umfassen, wobei in diesem Fall die Zahl der Einkoppelenden 91, 92 des Lichtleiters 90 vorzugsweise der Zahl der Konverterelemente entspricht.

Vorgesehen ist insbesondere mindestens eine lichtleitende Faser 100, welche das Licht des Lasers 10 auf die Fläche 175 der Konverterelemente 170, 171 lenkt. Auch hier entspricht vorzugsweise die Zahl der lichtleitenden Fasern 100 der Zahl der Konverterelemente 170, 171, wie hier beispielhaft für zwei Konverterelemente dargestellt. Weiterhin dargestellt ist das distale Ende 93 des Lichtleiters 90, wobei hier vorzugsweise eine Schnittstelle zu dem Lichtleiter 9 des zweiten Bauteils 5 vorgesehen sein kann.

### Bezugszeichenliste

- 1: Endoskop
- 3: Lichtquelle
- 5: zweites Bauteil, beispielsweise Schaft, des Endoskops
- 50: proximales Ende des zweiten Bauteils
- 51: distales Ende des zweiten Bauteils
- 53: Ummantelung
- 55: Einkoppelhülse
- 7: erstes Bauteil des Endoskops
- 9, 90: Lichtleiter
- 91, 92: Einkoppelenden des Lichtleiters 90
- 93: distales Ende des Lichtleiters 90, Schnittstelle
- 96: optisches Element, beispielsweise Linse
- 10: Laser
- 11, 100: lichtleitende Faser
- 12: Rücksignalleitung
- 15: Kamerachip
- 17: Konverter
- 170, 171: Konverterelement
- 172: Heat Sink, Wärmesenke
- 173, 174: Konvertermaterial
- 175: Fläche des Konverters
- 18: Daten- und/oder Bildverarbeitungseinheit

## Patentansprüche

1. Lichtquelle (3) für ein Endoskop (1), umfassend einen Laser (10) zur Abgabe von Primärlicht, vorzugsweise zu Abgabe von blauem und/oder ultravioletten Licht, sowie mindestens einen dem Laser zugeordneten Konverter (17) und einen Lichtleiter (90) mit einer oder mehreren lichtleitenden Fasern (11), wobei der Laser (10) so angeordnet ist, dass das Licht des Lasers (10) eine Fläche (175) des Konverters (17) zumindest teilweise bestrahlt und das proximale Ende des Lichtleiters (90) mit seiner mindestens einen lichtleitenden Faser (11) das konvertierte und/oder gestreute und/oder das reflektierte Licht des Konverters (17) aufnimmt,
**dadurch gekennzeichnet, dass** der Lichtleiter (90) am distalen Ende (93) eine Schnittstelle geeignet zur Ankopplung an einen weiteren Lichtleiter (9) aufweist.

2. Lichtquelle (3) nach Anspruch 1, umfassend eine lichtleitende Faser (100) zur Zuführung des Laserlichts zum Konverter (17), wobei das Auskoppelende der lichtleitenden Faser (100) und das Einkopplungsende des Lichtleiters (90) auf die gleiche Fläche (175) des Konverters (17) gerichtet sind, so dass der Konverter (17) in Remission betrieben wird und die Leitungsrichtung des Lichts in der lichtleitenden Faser (100) zur Leitung des Laserlichts entgegengesetzt zur Leitungsrichtung des vom Konverter (17) in den Lichtleiter (90) eingekoppelten Lichts ist.

3. Lichtquelle (3) nach einem der Ansprüche 1 oder 2, wobei der Konverter (17) zwei Konverterelemente (170, 171) umfasst, wobei die Konverterelemente (170, 171) jeweils ein keramisches Konvertermaterial (173, 174) enthalten, wobei vorzugsweise die Konverterelemente (170, 171) unterschiedliche Konvertermaterialien (173, 174) enthalten, so dass die Konverterelemente (170, 171) das Laserlicht in Licht unterschiedlicher spektraler Zusammensetzung konvertieren, wobei zumindest ein Laser (10) vorgesehen ist, welcher beide Konverterelemente jeweils mit einem Laserstrahl bestrahlt.

4. Lichtquelle (3) nach Anspruch 3, wobei der Lichtleiter (90) zwei Einkoppelenden (91, 92) aufweist, wobei die Konverterelemente (170, 171) so angeordnet sind, dass das von den Konverterelementen (170, 171) abgegebene Licht in jeweils eines der Einkoppelenden (91, 92) eingekoppelt wird.

5. Lichtquelle (3) nach einem der Ansprüche 3 oder 4, umfassend eine Einrichtung zur Einstellung des Verhältnisses der in den Lichtleiter eingekoppelten Lichtströme von beiden Konverterelemente (170, 171).

## Claims

1. A light source (3) for an endoscope (1), comprising a laser (10) for the emission of primary light, preferably for the emission of blue and/or ultraviolet light; and at least one converter (17) associated with said laser; and a light guide (90) comprising one or more optical fibres (11); wherein the laser (10) is arranged so that the light from the laser (10) is incident on at least a portion of a surface (175) of the converter (17) and so that the proximal end of the light guide (90) including its at least one optical fibre (11) receives the converted and/or scattered and/or reflected light from the converter (17);
**characterised in that** the light guide (90) has an interface at its distal end (93), which is suitable for being coupled to another light guide (9).

2. The light source (3) according to claim 1, comprising an optical fibre (100) for feeding the laser light to the converter (17), wherein the emission end of the optical fibre (100) and the injection end of the light guide (90) face the same surface (175) of the converter (17), such that the converter (17) is operated in remission and the direction of light conduction for conducting the laser light in the optical fibre (100) is opposite to the direction of conduction of the light injected into the light guide (90) from the converter (17).

3. The light source (3) according to any one of claims 1 or 2, wherein the converter (17) comprises two converter elements (170, 171), the converter elements (170, 171) each comprising a ceramic converter material (173, 174), wherein preferably the converter elements (170, 171) comprise different converter materials (173, 174) so that the converter elements (170, 171) convert the laser light into light of different spectral composition, wherein at least one laser (10) is provided for irradiating a respective laser beam onto the two converter elements.

4. The light source (3) according to claim 3, wherein the light guide (90) has two injection ends (91, 92), wherein the converter elements (170, 171) are arranged such that the light emitted by the converter elements (170, 171) is injected into a respective one of the injection ends (91, 92).

5. The light source (3) according to any one of claims 3 or 4, comprising a means for adjusting the ratio of the light fluxes injected into the light guide from the two converter elements (170, 171).

## Revendications

1. Source lumineuse (3) pour un endoscope (1), comprenant un laser (10) pour l'émission d'une lumière primaire, de préférence pour l'émission de la lumière bleue et/ou ultraviolette, ainsi qu'au moins un convertisseur (17) associé audit laser, et un guide de lumière (90) comprenant une ou plusieurs fibres optiques (11), le laser (10) étant disposé de sorte que la lumière du laser (10) irradie au moins partiellement une surface (175) du convertisseur (17) et que l'extrémité proximale du guide de lumière (90) avec sa ou ses fibres optiques (11) reçoit la lumière convertie et/ou diffusée et/ou réfléchie par le convertisseur (17),
chartérisée en ce que le guide de lumière (90) présente, à son extrémité distale (93), une interface apte à être couplée à un autre guide de lumière (9).

2. Source lumineuse (3) selon la revendication 1, comprenant une fibre optique (100) pour alimenter le convertisseur (17) en lumière laser, l'extrémité d'émission de la fibre optique (100) et l'extrémité d'injection du guide de lumière (90) faisant face à une même surface (175) du convertisseur (17), de sorte que le convertisseur (17) fonctionne en rémission, et que la direction de conduction de la lumière dans la fibre optique (100) pour conduire la lumière laser est opposée à la direction de conduction de la lumière injectée dans le guide de lumière (90) depuis le convertisseur (17).

3. Source lumineuse (3) selon l'une quelconque des revendications 1 ou 2, dans laquelle le convertisseur (17) comprend deux éléments convertisseurs (170, 171), les éléments convertisseurs (170, 171) comprenant chacun un matériau convertisseur céramique (173, 174), de préférence les éléments convertisseurs (170, 171) comprenant des matériaux convertisseurs différents (173, 174), de sorte que les éléments convertisseurs (170, 171) convertissent la lumière laser en lumière de composition spectrale différente, au moins un laser (10) étant prévu pour irradier un faisceau laser respectif sur les deux éléments convertisseurs.

4. Source lumineuse (3) selon la revendication 3, dans laquelle le guide de lumière (90) comprend deux extrémités d'injection (91, 92), les éléments convertisseurs (170, 171) étant disposés de telle sorte que la lumière émise par les éléments convertisseurs (170, 171) soit injectée respectivement dans l'une des extrémités d'injection (91, 92).

5. Source lumineuse (3) selon l'une quelconque des revendications 3 ou 4, comprenant un moyen de réglage du rapport des flux lumineux injectés dans le guide de lumière à partir des deux éléments convertisseurs (170, 171).
